# EUROPEAN PATENT SPECIFICATION

(11) **EP 3 830 101 B1**
(45) Date of publication and mention of the grant of the patent: **19.11.2025**
(21) Application number: 19744725.3
(22) Date of filing: 29.07.2019
(51) Int. Cl.: C07H 21/00, C07H 21/02, C07H 21/04, C07H 1/00, C07H 1/04, A61P 9/00

(54) **OLIGONUCLEOTIDES COMPRISING A PHOSPHOROTRITHIOATE INTERNUCLEOSIDE LINKAGE**
OLIGONUKLEOTIDE MIT EINER PHOSPHORDITHIOAT-INTERNUKLEOSID-VERKNÜPFUNG
OLIGONUCLÉOTIDES COMPRENANT UNE LIAISON INTERNUCLÉOSIDIQUE PHOSPHOROTRITHIOATE

(30) Priority: 31.07.2018 EP 18186677
(43) Date of publication of application: 09.06.2021
(73) Proprietor: Roche Innovation Center Copenhagen A/S, 2970 Hørsholm (DK)
(72) Inventor: BLEICHER, Konrad, 4070 Basel (CH); DUSCHMALÉ, Joerg Jakob Andreas, 4070 Basel (CH); DUSCHMALÉ, Martina Brigitte, 4070 Basel (CH); KOCH, Troels, 2970 Hørsholm (DK); KOLLER, Erich, 4070 Basel (CH); LI, Meiling, 4070 Basel (CH); SCHAEUBLIN, Adrian, 4070 Basel (CH)
(74) Representative: Pomeranc, Didier
(86) International application number: PCT/EP2019/070331
(87) International publication number: WO 2020/025527

(56) References cited:
- EP-A2- 3 491 002
- WO-A2-2018/019799
- US-A1- 2003 087 845
- MILLIGAN J F ET AL: "CURRENT CONCEPTS IN ANTISENSE DRUG DESIGN", JOURNAL OF MEDICINAL CHEMISTRY, AMERICAN CHEMICAL SOCIETY, US, vol. 36, no. 14, 9 July 1993 (1993-07-09), pages 1923 - 1937, XP002913877, ISSN: 0022-2623, DOI: 10.1021/JM00066A001

## Description

The invention relates to an oligonucleotide comprising at least one phosphorotrithioate internucleoside linkage of formula (I) 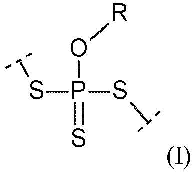 wherein one of the two bridging sulfur atoms is linked to the 3'carbon atom of a DNA nucleoside or a RNA nucleoside (A¹) and the other one is linked to the 5'carbon atom of another nucleoside (A²) and wherein R is hydrogen or a phosphate protecting group.

Short synthetic nucleic acids show a high potential as therapeutic agents. Since the first proof of concept studies in the late 1970ies, where unmodified deoxynucleotides were demonstrated to inhibit virus production *in vitro* (P. C. Zamecnik, M. L. Stephenson, Proc. Nat. Acad. Sci. USA 1978, 75, 280-284), remarkable progress has been achieved by their chemical modification. Due to its sensitivity towards nucleolytic degradation, the stabilization of the phosphodiester backbone of nucleic acids was an obvious starting point for chemical optimization of therapeutic oligonucleotides. As a consequence, a wide variety of phosphate modifications have been examined, including phosphorothioates (PS) (WO 2018/019799 A2 or

F. Eckstein, Antisense and Nucleic Acid Drug Development 2009, 10, 117-121.), phosphorodithioates (e.g. W. T. Weisler, M. H. Caruthers, J. Org. Chem 1996, 61, 4272-4281.), boranophosphates (e.g. J. S. Summers, B. R. Shaw, Curr. Med. Chem. 2001, 8, 1147-1155.), (thio)phosphoramidates (e.g. S. Gryaznov, T. Skorski, C. Cucco, M. Nieborowska-Skorska, C. Y. Chiu, D. Lloyd, J. Chen, M. Koziolkiewicz B. Calabretta, Nucleic Acids Res. 1996, 24, 1508-1514.) and methylphosphonates (e.g. P. S. Sarin, S. Agrawal, M. P. Civeira, J. Goodchild, T. Ikeuchi, P. C. Zamecnik, Proc. Nat. Acad. Sci. USA 1988, 20, 7448-7451). Among these phosphate analogues, arguably the most successful modification is the phosphorothioate, where one of the nonbridging phosphate oxygen atoms is replaced by sulfur. Due to their high stability towards nucleases and their pharmacokinetic benefits, phosphorothioate oligonucleotides became the first generation of oligonucleotide therapeutics and have paved the way for later generation modifications such as Locked Nucleic Acids (LNA) or 2'-O-(2-Methoxyethyl)-oligoribonucleotides (2'-MOE).

The present invention is directed to (thio)phosphate modifications wherein a phosphorotrithioate linkage is incorporated into oligonucleotide sequences. In phosphorotrithioates of this kind not only one of the nonbridging phosphate oxygen atom is replaced by sulfur but also the two bridging oxygen atoms in 3' and 5' positions of the adjacent ribose sugar rings.

We have surprisingly found that only one modification according to the invention can lead to an oligonucleotide with improved activity, both *in vitro* as well as *in vivo,* compared to the unmodified phosphorothioate analogue.
Figure 1 shows ApoB mRNA levels as well as intracellular oligonucleotide concentration (disposition) in primary rat hepatocytes at a treatment concentration of 1 µM after incubation times of 24 and 72 hrs.
Figure 2 shows ApoB mRNA levels in liver as well as kidney in C57BL/6JbomTac female mice treated with a 1 mg/kg single dose of an oligonucleotide bearing the modification according to the invention compared to a saline and an unmodified control at sacrifice on day 7.
Figure 3 shows serum cholesterol levels as measured in the blood of C57BL/6JbomTac female mice at day 0, 3 and 7 after a 1 mg/kg single dosing of an oligonucleotide bearing the modification according to the invention compared to a saline and an unmodified control.
Figure 4 shows oligonucleotide tissue content in liver, kidney and heart of C57BL/6JbomTac female mice treated with a 1 mg/kg single dose of an oligonucleotide bearing the modification according to the invention compared to an unmodified control at sacrifice on day 7.

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C₁-C₈ alkyl groups are methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl and propyl.

The term "cycloalkyl", alone or in combination, signifies a cycloalkyl ring with 3 to 8 carbon atoms and particularly a cycloalkyl ring with 3 to 6 carbon atoms. Examples of cycloalkyl are cyclopropyl, cyclobutyl, cyclopentyl, cyclohexyl, cycloheptyl and cyclooctyl, more particularly cyclopropyl and cyclobutyl. A particular example of "cycloalkyl" is cyclopropyl.

The term "alkoxy", alone or in combination, signifies a group of the formula alkyl-O- in which the term "alkyl" has the previously given significance, such as methoxy, ethoxy, n-propoxy, isopropoxy, n-butoxy, isobutoxy, sec.butoxy and tert.butoxy. Particular "alkoxy" are methoxy and ethoxy. Methoxyethoxy is a particular example of "alkoxyalkoxy".

The term "oxy", alone or in combination, signifies the -O- group.

The term "alkenyl", alone or in combination, signifies a straight-chain or branched hydrocarbon residue comprising an olefinic bond and up to 8, preferably up to 6, particularly preferred up to 4 carbon atoms. Examples of alkenyl groups are ethenyl, 1-propenyl, 2-propenyl, isopropenyl, 1-butenyl, 2-butenyl, 3-butenyl and isobutenyl.

The term "alkynyl", alone or in combination, signifies a straight-chain or branched hydrocarbon residue comprising a triple bond and up to 8, particularly 2 carbon atoms.

The terms "halogen" or "halo", alone or in combination, signifies fluorine, chlorine, bromine or iodine and particularly fluorine, chlorine or bromine, more particularly fluorine. The term "halo", in combination with another group, denotes the substitution of said group with at least one halogen, particularly substituted with one to five halogens, particularly one to four halogens, i.e. one, two, three or four halogens.

The term "haloalkyl", alone or in combination, denotes an alkyl group substituted with at least one halogen, particularly substituted with one to five halogens, particularly one to three halogens. Examples of haloalkyl include monofluoro-, difluoro- or trifluoromethyl, -ethyl or -propyl, for example 3,3,3-trifluoropropyl, 2-fluoroethyl, 2,2,2-trifluoroethyl, fluoromethyl or trifluoromethyl. Fluoromethyl, difluoromethyl and trifluoromethyl are particular "haloalkyl".

The term "halocycloalkyl", alone or in combination, denotes a cycloalkyl group as defined above substituted with at least one halogen, particularly substituted with one to five halogens, particularly one to three halogens. Particular example of "halocycloalkyl" are halocyclopropyl, in particular fluorocyclopropyl, difluorocyclopropyl and trifluorocyclopropyl.

The terms "hydroxyl" and "hydroxy", alone or in combination, signify the -OH group.

The terms "thiohydroxyl" and "thiohydroxy", alone or in combination, signify the - SH group.

The term "carbonyl", alone or in combination, signifies the -C(O)- group.

The term "carboxy" or "carboxyl", alone or in combination, signifies the -COOH group.

The term "amino", alone or in combination, signifies the primary amino group (-NH₂), the secondary amino group (-NH-), or the tertiary amino group (-N-).

The term "alkylamino", alone or in combination, signifies an amino group as defined above substituted with one or two alkyl groups as defined above.

The term "sulfonyl", alone or in combination, means the -SO₂ group.

The term "sulfinyl", alone or in combination, signifies the -SO- group.

The term "sulfanyl", alone or in combination, signifies the -S- group.

The term "cyano", alone or in combination, signifies the -CN group.

The term "azido", alone or in combination, signifies the -N₃ group.

The term "nitro", alone or in combination, signifies the NO₂ group.

The term "formyl", alone or in combination, signifies the -C(O)H group.

The term "carbamoyl", alone or in combination, signifies the -C(O)NH₂ group.

The term "cabamido", alone or in combination, signifies the -NH-C(O)-NH₂ group.

The term "aryl", alone or in combination, denotes a monovalent aromatic carbocyclic mono- or bicyclic ring system comprising 6 to 10 carbon ring atoms, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples of aryl include phenyl and naphthyl, in particular phenyl.

The term "heteroaryl", alone or in combination, denotes a monovalent aromatic heterocyclic mono- or bicyclic ring system of 5 to 12 ring atoms, comprising 1, 2, 3 or 4 heteroatoms selected from N, O and S, the remaining ring atoms being carbon, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples of heteroaryl include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, quinoxalinyl, carbazolyl or acridinyl.

The term "heterocyclyl", alone or in combination, signifies a monovalent saturated or partly unsaturated mono- or bicyclic ring system of 4 to 12, in particular 4 to 9 ring atoms, comprising 1, 2, 3 or 4 ring heteroatoms selected from N, O and S, the remaining ring atoms being carbon, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples for monocyclic saturated heterocyclyl are azetidinyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl or dihydropyranyl.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The oligonucleotide of the invention can also be present in the form of zwitterions. Particularly preferred pharmaceutically acceptable salts of the invention are the sodium, lithium, potassium and trialkylammonium salts.

The term "protecting group", alone or in combination, signifies a group which selectively blocks a reactive site in a multifunctional compound such that a chemical reaction can be carried out selectively at another unprotected reactive site. Protecting groups can be removed. Exemplary protecting groups are amino-protecting groups, carboxy-protecting groups or hydroxy-protecting groups.

"Phosphate protecting group" is a protecting group of the phosphate group. Examples of phosphate protecting group are 2-cyanoethyl and methyl. A particular example of phosphate protecting group is 2-cyanoethyl.

"Hydroxyl protecting group" is a protecting group of the hydroxyl group and is also used to protect thiol groups. Examples of hydroxyl protecting groups are acetyl (Ac), benzoyl (Bz), benzyl (Bn), β-methoxyethoxymethyl ether (MEM), dimethoxytrityl (or bis-(4-methoxyphenyl)phenylmethyl) (DMT), trimethoxytrityl (or tris-(4-methoxyphenyl)phenylmethyl) (TMT), methoxymethyl ether (MOM), methoxytrityl [(4-methoxyphenyl)diphenylmethyl (MMT), p-methoxybenzyl ether (PMB), methylthiomethyl ether, pivaloyl (Piv), tetrahydropyranyl (THP), tetrahydrofuran (THF), trityl or triphenylmethyl (Tr), silyl ether (for example trimethylsilyl (TMS), tertbutyldimethylsilyl (TBDMS), tri-iso-propylsilyloxymethyl (TOM) and triisopropylsilyl (TIPS) ethers), methyl ethers and ethoxyethyl ethers (EE). Particular examples of hydroxyl protecting group are DMT and TMT, in particular DMT.

"Thiohydroxyl protecting group" is a protecting group of the thiohydroxyl group. Examples of thiohydroxyl protecting groups are those of the "hydroxyl protecting group".

If one of the starting materials or compounds of the invention contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compounds described herein can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

### Oligonucleotide

The term "oligonucleotide" as used herein is defined as it is generally understood by the skilled person as a molecule comprising two or more covalently linked nucleosides. Such covalently bound nucleosides may also be referred to as nucleic acid molecules or oligomers. Oligonucleotides are commonly made in the laboratory by solid-phase chemical synthesis followed by purification. When referring to a sequence of the oligonucleotide, reference is made to the sequence or order of nucleobase moieties, or modifications thereof, of the covalently linked nucleotides or nucleosides. The oligonucleotide of the invention is man-made, and is chemically synthesized, and is typically purified or isolated. The oligonucleotide of the invention may comprise one or more modified nucleosides or nucleotides.

### Antisense oligonucleotides

The term "Antisense oligonucleotide" as used herein is defined as oligonucleotides capable of modulating expression of a target gene by hybridizing to a target nucleic acid, in particular to a contiguous sequence on a target nucleic acid. The antisense oligonucleotides are not essentially double stranded and are therefore not siRNAs or shRNAs. Preferably, the antisense oligonucleotides of the present invention are single stranded. It is understood that single stranded oligonucleotides of the present invention can form hairpins or intermolecular duplex structures (duplex between two molecules of the same oligonucleotide), as long as the degree of intra or inter self complementarity is less than 50% across of the full length of the oligonucleotide

### Contiguous Nucleotide Sequence

The term "contiguous nucleotide sequence" refers to the region of the oligonucleotide which is complementary to the target nucleic acid. The term is used interchangeably herein with the term "contiguous nucleobase sequence" and the term "oligonucleotide motif sequence". In some embodiments all the nucleotides of the oligonucleotide constitute the contiguous nucleotide sequence. In some embodiments the oligonucleotide comprises the contiguous nucleotide sequence, such as a F-G-F' gapmer region, and may optionally comprise further nucleotide(s), for example a nucleotide linker region which may be used to attach a functional group to the contiguous nucleotide sequence. The nucleotide linker region may or may not be complementary to the target nucleic acid.

### Nucleotides

Nucleotides are the building blocks of oligonucleotides and polynucleotides, and for the purposes of the present invention include both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

### Modified nucleoside

The term "modified nucleoside" or "nucleoside modification" as used herein refers to nucleosides modified as compared to the equivalent DNA or RNA nucleoside by the introduction of one or more modifications of the sugar moiety or the (nucleo)base moiety. In a preferred embodiment the modified nucleoside comprise a modified sugar moiety. The term modified nucleoside may also be used herein interchangeably with the term "nucleoside analogue" or modified "units" or modified "monomers". Nucleosides with an unmodified DNA or RNA sugar moiety are termed DNA or RNA nucleosides herein. Nucleosides with modifications in the base region of the DNA or RNA nucleoside are still generally termed DNA or RNA if they allow Watson Crick base pairing.

### Modified internucleoside linkage

The term "modified internucleoside linkage" is defined as generally understood by the skilled person as linkages other than phosphodiester (PO) linkages, that covalently couples two nucleosides together. The oligonucleotides of the invention may therefore comprise modified internucleoside linkages. In some embodiments, the modified internucleoside linkage increases the nuclease resistance of the oligonucleotide compared to a phosphodiester linkage. For naturally occurring oligonucleotides, the internucleoside linkage includes phosphate groups creating a phosphodiester bond between adjacent nucleosides. Modified internucleoside linkages are particularly useful in stabilizing oligonucleotides for *in vivo* use, and may serve to protect against nuclease cleavage at regions of DNA or RNA nucleosides in the oligonucleotide of the invention, for example within the gap region of a gapmer oligonucleotide, as well as in regions of modified nucleosides, such as region F and F'.

In an embodiment, the oligonucleotide comprises one or more internucleoside linkages modified from the natural phosphodiester, such one or more modified internucleoside linkages that is for example more resistant to nuclease attack. Nuclease resistance may be determined by incubating the oligonucleotide in blood serum or by using a nuclease resistance assay (e.g. snake venom phosphodiesterase (SVPD)), both are well known in the art. Internucleoside linkages which are capable of enhancing the nuclease resistance of an oligonucleotide are referred to as nuclease resistant internucleoside linkages. In some embodiments at least 50% of the internucleoside linkages in the oligonucleotide, or contiguous nucleotide sequence thereof, are modified, such as at least 60%, such as at least 70%, such as at least 80 or such as at least 90% of the internucleoside linkages in the oligonucleotide, or contiguous nucleotide sequence thereof, are nuclease resistant internucleoside linkages. In some embodiments all of the internucleoside linkages of the oligonucleotide, or contiguous nucleotide sequence thereof, are nuclease resistant internucleoside linkages. It will be recognized that, in some embodiments the nucleosides which link the oligonucleotide of the invention to a non-nucleotide functional group, such as a conjugate, may be phosphodiester.

A preferred modified internucleoside linkage for use in the oligonucleotide of the invention is phosphorothioate.

Phosphorothioate internucleoside linkages are particularly useful due to nuclease resistance, beneficial pharmacokinetics and ease of manufacture. In some embodiments at least 50% of the internucleoside linkages in the oligonucleotide, or contiguous nucleotide sequence thereof, are phosphorothioate, such as at least 60%, such as at least 70%, such as at least 80% or such as at least 90% of the internucleoside linkages in the oligonucleotide, or contiguous nucleotide sequence thereof, are phosphorothioate. In some embodiments, other than the phosphorotrithioate internucleoside linkages, all of the internucleoside linkages of the oligonucleotide, or contiguous nucleotide sequence thereof, are phosphorothioate. In some embodiments, the oligonucleotide of the invention comprises both phosphorothioate internucleoside linkages and at least one phosphodiester linkage, such as 2, 3 or 4 phosphodiester linkages, in addition to the phosphorotrithioate linkage(s). In a gapmer oligonucleotide, phosphodiester linkages, when present, are suitably not located between contiguous DNA nucleosides in the gap region G.

Nuclease resistant linkages, such as phosphorothioate linkages, are particularly useful in oligonucleotide regions capable of recruiting nuclease when forming a duplex with the target nucleic acid, such as region G for gapmers. Phosphorothioate linkages may, however, also be useful in non-nuclease recruiting regions and/or affinity enhancing regions such as regions F and F' for gapmers. Gapmer oligonucleotides may, in some embodiments comprise one or more phosphodiester linkages in region F or F', or both region F and F', which the internucleoside linkage in region G may be fully phosphorothioate.

Advantageously, all the internucleoside linkages in the contiguous nucleotide sequence of the oligonucleotide, or all the internucleoside linkages of the oligonucleotide, are phosphorothioate linkages.

It is recognized that, as disclosed in EP 2 742 135, antisense oligonucleotides may comprise other internucleoside linkages (other than phosphodiester and phosphorothioate), for example alkyl phosphonate/methyl phosphonate internucleosides, which according to EP 2 742 135 may for example be tolerated in an otherwise DNA phosphorothioate the gap region.

### Stereorandom Phosphorothioate Linkages

Phosphorothioate linkages are internucleoside phosphate linkages where one of the non-bridging oxygens has been substituted with a sulfur. The substitution of one of the non-bridging oxygens with a sulfur introduces a chiral center, and as such within a single phosphorothioate oligonucleotide, each phosphorothioate internucleoside linkage will be either in the S (Sp) or R (Rp) stereoisoforms. Such internucleoside linkages are referred to as "chiral internucleoside linkages". By comparison, phosphodiester internucleoside linkages are non-chiral as they have two non-terminal oxygen atoms.

The designation of the chirality of a stereocenter is determined by standard Cahn-Ingold-Prelog rules (CIP priority rules) first published in Cahn, R.S.; Ingold, C.K.; Prelog, V. (1966) "Specification of Molecular Chirality" Angewandte Chemie International Edition 5 (4): 385-415. doi: 10.1002/anie.196603851.

During standard oligonucleotide synthesis the stereoselectivity of the coupling and the following sulfurization is not controlled. For this reason the stereochemistry of each phosphorothioate internucleoside linkages is randomly Sp or Rp, and as such a phosphorothioate oligonucleotide produced by traditional oligonucleotide synthesis actually can exist in as many as 2^{X} different phosphorothioate diastereoisomers, where X is the number of phosphorothioate internucleoside linkages. Such oligonucleotides are referred to as stereorandom phosphorothioate oligonucleotides herein, and do not contain any stereodefined internucleoside linkages. Stereorandom phosphorothioate oligonucleotides are therefore mixtures of individual diastereoisomers originating from the non-stereodefined synthesis. In this context the mixture is defined as up to 2^{X} different phosphorothioate diastereoisomers.

### Stereodefined Internucleoside Linkages

A stereodefined internucleoside linkage is a chiral internucleoside linkage having a diastereoisomeric excess for one of its two diastereomeric forms, Rp or Sp.

It should be recognized that stereoselective oligonucleotide synthesis methods used in the art typically provide at least about 90% or at least about 95% diastereoselectivity at each chiral internucleoside linkage, and as such up to about 10%, such as about 5% of oligonucleotide molecules may have the alternative diastereoisomeric form.

In some embodiments the diastereoisomeric ratio of each stereodefined chiral internucleoside linkage is at least about 90:10. In some embodiments the diastereoisomeric ratio of each chiral internucleoside linkage is at least about 95:5.

The stereodefined phosphorothioate linkage is a particular example of stereodefined internucleoside linkage.

### Stereodefined phosphorothioate linkage

A stereodefined phosphorothioate linkage is a phosphorothioate linkage having a diastereomeric excess for one of its two diastereosiomeric forms, Rp or Sp.

The Rp and Sp configurations of the phosphorothioate internucleoside linkages are presented below

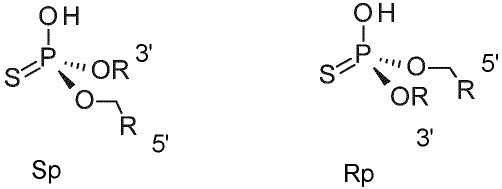

Where the 3' R group represents the 3' position of the adjacent nucleoside (a 5' nucleoside), and the 5' R group represents the 5' position of the adjacent nucleoside (a 3' nucleoside).

Rp internucleoside linkages may also be represented as srP, and Sp internucleoside linkages may be represented as ssP herein.

In a particular embodiment, the diastereomeric ratio of each stereodefined phosphorothioate linkage is at least about 90:10 or at least 95:5.

In some embodiments the diastereomeric ratio of each stereodefined phosphorothioate linkage is at least about 97:3. In some embodiments the diastereomeric ratio of each stereodefined phosphorothioate linkage is at least about 98:2. In some embodiments the diastereomeric ratio of each stereodefined phosphorothioate linkage is at least about 99: 1.

In some embodiments a stereodefined internucleoside linkage is in the same diastereomeric form (Rp or Sp) in at least 97%, such as at least 98%, such as at least 99%, or (essentially) all of the oligonucleotide molecules present in a population of the oligonucleotide molecule.

Diastereomeric purity can be measured in a model system only having an achiral backbone (*i.e.* phosphodiesters). It is possible to measure the diastereomeric purity of each monomer by e.g. coupling a monomer having a stereodefine internucleoside linkage to the following model-system "5' t-po-t-po-t-po 3'". The result of this will then give : 5' DMTr-t-srp-t-po-t-po-t-po 3' or 5' DMTr-t-ssp-t-po-t-po-t-po 3' which can be separated using HPLC. The diastereomeric purity is determined by integrating the UV signal from the two possible diastereoisomers and giving a ratio of these e.g. 98:2, 99:1 or >99:1.

It will be understood that the diastereomeric purity of a specific single diastereoisomer (a single stereodefined oligonucleotide molecule) will be a function of the coupling selectivity for the defined stereocenter at each internucleoside position, and the number of stereodefined internucleoside linkages to be introduced. By way of example, if the coupling selectivity at each position is 97%, the resulting purity of the stereodefined oligonucleotide with 15 stereodefined internucleoside linkages will be 0.97¹⁵, *i.e.* 63% of the desired diastereoisomer as compared to 37% of the other diastereoisomers. The purity of the defined diastereoisomer may after synthesis be improved by purification, for example by HPLC, such as ion exchange chromatography or reverse phase chromatography.

In some embodiments, a stereodefined oligonucleotide refers to a population of an oligonucleotide wherein at least about 40%, such as at least about 50% of the population is of the desired diastereoisomer.

Alternatively stated, in some embodiments, a stereodefined oligonucleotide refers to a population of oligonucleotides wherein at least about 40%, such as at least about 50%, of the population consists of the desired (specific) stereodefined internucleoside linkage motifs (also termed stereodefined motif).

For stereodefined oligonucleotides which comprise both stereorandom and stereodefined internucleoside chiral centers, the purity of the stereodefined oligonucleotide is determined with reference to the % of the population of the oligonucleotide which retains the desired stereodefined internucleoside linkage motif(s), the stereorandom linkages being disregarded in the calculation.

### Nucleobase

The term nucleobase includes the purine (e.g. adenine and guanine) and pyrimidine (e.g. uracil, thymine and cytosine) moieties present in nucleosides and nucleotides which form hydrogen bonds in nucleic acid hybridization. In the context of the present invention the term nucleobase also encompasses modified nucleobases which may differ from naturally occurring nucleobases, but are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of Chemical Research vol 45 page 2055 and Bergstrom (2009) Current Protocols in Nucleic Acid Chemistry Suppl. 37 1.4.1.

In some embodiments the nucleobase moiety is modified by changing the purine or pyrimidine into a modified purine or pyrimidine, such as substituted purine or substituted pyrimidine, such as a nucleobase selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine and 2-chloro-6-aminopurine.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are selected from A, T, G, C, and 5-methyl cytosine. Optionally, for LNA gapmers, 5-methyl cytosine LNA nucleosides may be used.

### Modified oligonucleotide

The term modified oligonucleotide describes an oligonucleotide comprising one or more sugar-modified nucleosides and/or modified internucleoside linkages. The term chimeric" oligonucleotide is a term that has been used in the literature to describe oligonucleotides with modified nucleosides.

### Stereodefined oligonucleotide

A stereodefined oligonucleotide is an oligonucleotide wherein at least one of the internucleoside linkages is a stereodefined internucleoside linkage.

A stereodefined phosphorothioate oligonucleotide is an oligonucleotide wherein at least one of the internucleoside linkages is a stereodefined phosphorothioate internucleoside linkage.

### Complementarity

The term "complementarity" describes the capacity for Watson-Crick base-pairing of nucleosides/nucleotides. Watson-Crick base pairs are guanine (G)-cytosine (C) and adenine (A) - thymine (T)/uracil (U). It will be understood that oligonucleotides may comprise nucleosides with modified nucleobases, for example 5-methyl cytosine is often used in place of cytosine, and as such the term complementarity encompasses Watson Crick base-paring between non-modified and modified nucleobases (see for example Hirao et al (2012) Accounts of Chemical Research vol 45 page 2055 and Bergstrom (2009) Current Protocols in Nucleic Acid Chemistry Suppl. 37 1.4.1).

The term "% complementary" as used herein, refers to the proportion of nucleotides in a contiguous nucleotide sequence in a nucleic acid molecule (e.g. oligonucleotide) which, at a given position, are complementary to (*i.e.* form Watson Crick base pairs with) a contiguous nucleotide sequence, at a given position of a separate nucleic acid molecule (e.g. the target nucleic acid). The percentage is calculated by counting the number of aligned bases that form pairs between the two sequences (when aligned with the target sequence 5'-3' and the oligonucleotide sequence from 3'-5'), dividing by the total number of nucleotides in the oligonucleotide and multiplying by 100. In such a comparison a nucleobase/nucleotide which does not align (form a base pair) is termed a mismatch. Preferably, insertions and deletions are not allowed in the calculation of % complementarity of a contiguous nucleotide sequence.

The term "fully complementary", refers to 100% complementarity.

### Identity

The term "Identity" as used herein, refers to the number of nucleotides in percent of a contiguous nucleotide sequence in a nucleic acid molecule (e.g. oligonucleotide) which, at a given position, are identical to (*i.e.* in their ability to form Watson Crick base pairs with the complementary nucleoside) a contiguous nucleotide sequence, at a given position of a separate nucleic acid molecule (e.g. the target nucleic acid). The percentage is calculated by counting the number of aligned bases that are identical between the two sequences dividing by the total number of nucleotides in the oligonucleotide and multiplying by 100. Percent Identity = (Matches x 100)/Length of aligned region. Preferably, insertions and deletions are not allowed in the calculation of % complementarity of a contiguous nucleotide sequence.

### Hybridization

The term "hybridizing" or "hybridizes" as used herein is to be understood as two nucleic acid strands (e.g. an oligonucleotide and a target nucleic acid) forming hydrogen bonds between base pairs on opposite strands thereby forming a duplex. The affinity of the binding between two nucleic acid strands is the strength of the hybridization. It is often described in terms of the melting temperature (Tₘ) defined as the temperature at which half of the oligonucleotides are duplexed with the target nucleic acid. At physiological conditions Tₘ is not strictly proportional to the affinity (Mergny and Lacroix, 2003,Oligonucleotides 13:515-537). The standard state Gibbs free energy ΔG° is a more accurate representation of binding affinity and is related to the dissociation constant (K_{d}) of the reaction by ΔG°=-RTln(K_{d}), where R is the gas constant and T is the absolute temperature. Therefore, a very low ΔG° of the reaction between an oligonucleotide and the target nucleic acid reflects a strong hybridization between the oligonucleotide and target nucleic acid. ΔG° is the energy associated with a reaction where aqueous concentrations are 1M, the pH is 7, and the temperature is 37°C. The hybridization of oligonucleotides to a target nucleic acid is a spontaneous reaction and for spontaneous reactions ΔG° is less than zero. ΔG° can be measured experimentally, for example, by use of the isothermal titration calorimetry (ITC) method as described in Hansen et al., 1965, Chem. Comm. 36-38 and Holdgate et al., 2005, Drug Discov Today*.* The skilled person will know that commercial equipment is available for ΔG° measurements. ΔG° can also be estimated numerically by using the nearest neighbor model as described by SantaLucia, 1998, Proc Natl Acad Sci USA. 95: 1460-1465 using appropriately derived thermodynamic
parameters described by Sugimoto et al., 1995, Biochemistry 34:11211-11216 and McTigue et al., 2004, Biochemistry 43:5388-5405. In order to have the possibility of modulating its intended nucleic acid target by hybridization, oligonucleotides of the present invention hybridize to a target nucleic acid with estimated ΔG° values below -10 kcal for oligonucleotides that are 10-30 nucleotides in length. In some embodiments the degree or strength of hybridization is measured by the standard state Gibbs free energy ΔG°. The oligonucleotides may hybridize to a target nucleic acid with estimated ΔG° values below the range of -10 kcal, such as below -15 kcal, such as below -20 kcal and such as below -25 kcal for oligonucleotides that are 8-30 nucleotides in length. In some embodiments the oligonucleotides hybridize to a target nucleic acid with an estimated ΔG° value of -10 to -60 kcal, such as -12 to -40, such as from -15 to -30 kcal or-16 to -27 kcal such as -18 to -25 kcal.

### Sugar modifications

The oligomer of the invention may comprise one or more nucleosides which have a modified sugar moiety, *i.e.* a modification of the sugar moiety when compared to the ribose sugar moiety found in DNA and RNA.

Numerous nucleosides with modification of the ribose sugar moiety have been made, primarily with the aim of improving certain properties of oligonucleotides, such as affinity and/or nuclease resistance.

Such modifications include those where the ribose ring structure is modified, e.g. by replacement with a hexose ring (HNA), or a bicyclic ring, which typically have a biradical bridge between the C2 and C4 carbons on the ribose ring (LNA), or an unlinked ribose ring which typically lacks a bond between the C2 and C3 carbons (e.g. UNA). Other sugar modified nucleosides include, for example, bicyclohexose nucleic acids (WO 2011/017521) or tricyclic nucleic acids (WO 2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example be introduced at the 2', 3', 4' or 5' positions.

### 2' sugar modified nucleosides.

A 2' sugar modified nucleoside is a nucleoside which has a substituent other than H or -OH at the 2' position (2' substituted nucleoside) or comprises a 2' linked biradical capable of forming a bridge between the 2' carbon and a second carbon in the ribose ring, such as LNA (2' - 4' biradical bridged) nucleosides.

Indeed, much focus has been spent on developing 2' substituted nucleosides, and numerous 2' substituted nucleosides have been found to have beneficial properties when incorporated into oligonucleotides. For example, the 2' modified sugar may provide enhanced binding affinity and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-fluoro-RNA and 2'-F-ANA nucleoside. Further examples can be found in e.g. Freier & Altmann; Nucl. Acid Res., 1997, 25, 4429-4443 and Uhlmann; Curr. Opinion in Drug Development, 2000, 3(2), 293-213 and Deleavey and Damha, Chemistry and Biology 2012, 19, 937. Below are illustrations of some 2' substituted modified nucleosides.

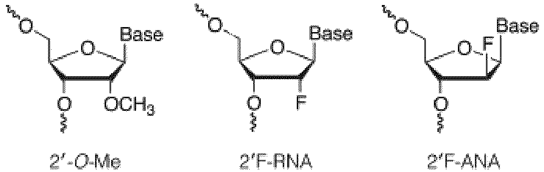

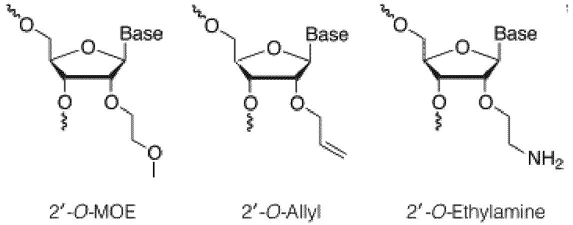

In relation to the present invention 2' substituted does not include 2' bridged molecules like LNA.

### Locked Nucleic Acid Nucleosides (LNA nucleosides)

A "LNA nucleoside" is a 2'-modified nucleoside which comprises a biradical linking the C2' and C4' of the ribose sugar ring of said nucleoside (also referred to as a "2'- 4' bridge"), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352 , WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al. J. Org. Chem. 2010, Vol 75(5) pp. 1569-81 and Mitsuoka et al., Nucleic Acids Research 2009, 37(4), 1225-1238.

The 2'-4' bridge comprises 2 to 4 bridging atoms and is in particular of formula -X-Y-, X being linked to C4' and Y linked to C2', wherein
X is oxygen, sulfur, -CR^{a}R^{b}-, -C(R^{a})=C(R^{b})-, -C(=CR^{a}R^{b})-, -C(R^{a})=N-, -Si(R^{a})₂-, - SO2-, -NR^{a}-; -O-NR^{a}-, -NR^{a}-O-, -C(=J)-, Se, -O-NR^{a}-, -NR^{a}-CR^{a}R^{b}-, -N(R^{a})-O- or -O-CR^{a}R^{b}-;
Y is oxygen, sulfur, -(CR^{a}R^{b})ₙ-, -CR^{a}R^{b}-O-CR^{a}R^{b}-, -C(R^{a})=C(R^{b})-, -C(R^{a})=N-, - Si(R^{a})2-, -SO₂-, -NR^{a}-, -C(=J)-, Se, -O-NR^{a}-, -NR^{a}-CR^{a}R^{b}-, -N(R^{a})-O- or -O-CR^{a}R^{b}-;
with the proviso that -X-Y- is not -O-O-, Si(R^{a})₂-Si(R^{a})₂-, -SO₂-SO₂-, -C(R^{a})=C(R^{b})-C(R^{a})=C(R^{b}), -C(R^{a})=N-C(R^{a})=N-, -C(R^{a})=N-C(R^{a})=C(R^{b}) , -C(R^{a})=C(^{Rb})-C(R^{a})=N- or -Se-Se-;
J is oxygen, sulfur, =CH₂ or =N(R^{a});
R^{a} and R^{b} are independently selected from hydrogen, halogen, hydroxyl, cyano, thiohydroxyl, alkyl, substituted alkyl, alkenyl, substituted alkenyl, alkynyl, substituted alkynyl, alkoxy, substituted alkoxy, alkoxyalkyl, alkenyloxy, carboxyl, alkoxycarbonyl, alkylcarbonyl, formyl, aryl, heterocyclyl, amino, alkylamino, carbamoyl, alkylaminocarbonyl, aminoalkylaminocarbonyl, alkylaminoalkylaminocarbonyl, alkylcarbonylamino, carbamido, alkanoyloxy, sulfonyl, alkylsulfonyloxy, nitro, azido, thiohydroxylsulfidealkylsulfanyl, aryloxycarbonyl, aryloxy, arylcarbonyl, heteroaryl, heteroaryloxycarbonyl, heteroaryloxy, heteroarylcarbonyl, -OC(=X^{a})R^{c}, -OC(=X^{a})NR^{c}R^{d} and - NR^{e}C(=X^{a})NR^{c}R^{d};
or two geminal R^{a} and R^{b} together form optionally substituted methylene;
or two geminal R^{a} and R^{b}, together with the carbon atom to which they are attached, form cycloalkyl or halocycloalkyl, with only one carbon atom of -X-Y-;
wherein substituted alkyl, substituted alkenyl, substituted alkynyl, substituted alkoxy and substituted methylene are alkyl, alkenyl, alkynyl and methylene substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl, formyl, heterocylyl, aryl and heteroaryl;
X^{a} is oxygen, sulfur or -NR^{c};
R^{c}, R^{d} and R^{e} are independently selected from hydrogen and alkyl; and
n is 1, 2 or 3.

In a further particular embodiment of the invention, X is oxygen, sulfur, -NR^{a}-, - CR^{a}R^{b}- or -C(=CR^{a}R^{b})-, particularly oxygen, sulfur, -NH-, -CH₂- or -C(=CH₂)-, more particularly oxygen.

In another particular embodiment of the invention, Y is -CR^{a}R^{b}-, -CR^{a}R^{b}-CR^{a}R^{b}- or -CR^{a}R^{b-}CR^{a}R^{b-}CR^{a}R^{b}-, particularly -CH₂-CHCH₃-, -CHCH₃-CH₂-, -CH₂-CH₂- or -CH₂-CH₂-CH₂-.

In a particular embodiment of the invention, -X-Y- is -O-(CR^{a}R^{b})ₙ-, -S-CR^{a}R^{b}-, - N(R^{a})CR^{a}R^{b}-, -CR^{a}R^{b}-CR^{a}R^{b}-, -O-CR^{a}R^{b}-O-CR^{a}R^{b}-, -CR^{a}R^{b}-O-CR^{a}R^{b}-, -C(=CR^{a}R^{b})-CR^{a}R^{b}-, -N(R^{a})CR^{a}R^{b}-, -O-N(R^{a})-CR^{a}R^{b}- or -N(R^{a})-O-CR^{a}R^{b}-.

In a particular embodiment of the invention, R^{a} and R^{b} are independently selected from the group consisting of hydrogen, halogen, hydroxyl, alkyl and alkoxyalkyl, in particular hydrogen, halogen, alkyl and alkoxyalkyl.

In another embodiment of the invention, R^{a} and R^{b} are independently selected from the group consisting of hydrogen, fluoro, hydroxyl, methyl and -CH₂-O-CH₃, in particular hydrogen, fluoro, methyl and -CH₂-O-CH₃.

Advantageously, one of R^{a} and R^{b} of -X-Y- is as defined above and the other ones are all hydrogen at the same time.

In a further particular embodiment of the invention, R^{a} is hydrogen or alkyl, in particular hydrogen or methyl.

In another particular embodiment of the invention, R^{b} is hydrogen or or alkyl, in particular hydrogen or methyl.

In a particular embodiment of the invention, one or both of R^{a} and R^{b} are hydrogen.

In a particular embodiment of the invention, only one of R^{a} and R^{b} is hydrogen.

In one particular embodiment of the invention, one of R^{a} and R^{b} is methyl and the other one is hydrogen.

In a particular embodiment of the invention, R^{a} and R^{b} are both methyl at the same time.

In a particular embodiment of the invention, -X-Y- is -O-CH₂-, -S-CH₂-, -S-CH(CH₃)-, -NH-CH₂-, -O-CH₂CH₂-, -O-CH(CH₂-O-CH₃)-, -O-CH(CH₂CH₃)-, -O-CH(CH₃)-, -O-CH₂-O-CH₂-, -O-CH₂-O-CH₂-, -CH₂-O-CH₂-, -C(=CH₂)CH₂-, - C(=CH₂)CH(CH₃)-, -N(OCH₃)CH₂- or -N(CH₃)CH₂-;

In a particular embodiment of the invention, -X-Y- is -O-CR^{a}R^{b}-wherein R^{a} and R^{b} are independently selected from the group consisting of hydrogen, alkyl and alkoxyalkyl, in particular hydrogen, methyl and -CH₂-O-CH₃.

In a particular embodiment, -X-Y- is -O-CH₂- or -O-CH(CH₃)-, particularly -O-CH₂-.

The 2'- 4' bridge may be positioned either below the plane of the ribose ring (beta-D- configuration), or above the plane of the ring (alpha-L- configuration), as illustrated in formula (A) and formula (B) respectively.

The LNA nucleoside according to the invention is in particular of formula (B 1) or (B2) 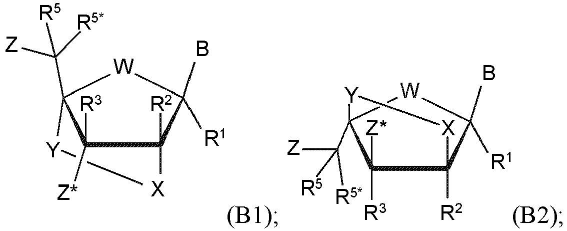 wherein
W is oxygen, sulfur, -N(R^{a})- or -CR^{a}R^{b}-, in particular oxygen;
B is a nucleobase or a modified nucleobase;
Z is an internucleoside linkage to an adjacent nucleoside or a 5'-terminal group;
Z* is an internucleoside linkage to an adjacent nucleoside or a 3'-terminal group;
R¹, R², R³, R⁵ and R⁵* are independently selected from hydrogen, halogen, alkyl, haloalkyl, alkenyl, alkynyl, hydroxy, alkoxy, alkoxyalkyl, azido, alkenyloxy, carboxyl, alkoxycarbonyl, alkylcarbonyl, formyl and aryl; and
X, Y, R^{a} and R^{b} are as defined above.

In a particuliar embodiment, in the definition of -X-Y-, R^{a} is hydrogen or alkyl, in particular hydrogen or methyl. In another particular embodiment, in the definition of -X-Y-, R^{b} is hydrogen or alkyl, in particular hydrogen or methyl. In a further particular embodiment, in the definition of -X-Y-, one or both of R^{a} and R^{b} are hydrogen. In a particular embodiment, in the definition of -X-Y-, only one of R^{a} and R^{b} is hydrogen. In one particular embodiment, in the definition of -X-Y-, one of R^{a} and R^{b} is methyl and the other one is hydrogen. In a particular embodiment, in the definition of -X-Y-, R^{a} and R^{b} are both methyl at the same time.

In a further particuliar embodiment, in the definition of X, R^{a} is hydrogen or alkyl, in particular hydrogen or methyl. In another particular embodiment, in the definition of X, R^{b} is hydrogen or alkyl, in particular hydrogen or methyl. In a particular embodiment, in the definition of X, one or both of R^{a} and R^{b} are hydrogen. In a particular embodiment, in the definition of X, only one of R^{a} and R^{b} is hydrogen. In one particular embodiment, in the definition of X, one of R^{a} and R^{b} is methyl and the other one is hydrogen. In a particular embodiment, in the definition of X, R^{a} and R^{b} are both methyl at the same time.

In a further particuliar embodiment, in the definition of Y, R^{a} is hydrogen or alkyl, in particular hydrogen or methyl. In another particular embodiment, in the definition of Y, R^{b} is hydrogen or alkyl, in particular hydrogen or methyl. In a particular embodiment, in the definition of Y, one or both of R^{a} and R^{b} are hydrogen. In a particular embodiment, in the definition of Y, only one of R^{a} and R^{b} is hydrogen. In one particular embodiment, in the definition of Y, one of R^{a} and R^{b} is methyl and the other one is hydrogen. In a particular embodiment, in the definition of Y, R^{a} and R^{b} are both methyl at the same time.

In a particular embodiment of the invention R¹, R², R³, R⁵ and R⁵* are independently selected from hydrogen and alkyl, in particular hydrogen and methyl.

In a further particular advantageous embodiment of the invention, R¹, R², R³, R⁵ and R⁵* are all hydrogen at the same time.

In another particular embodiment of the invention, R¹, R², R³, are all hydrogen at the same time, one of R⁵ and R⁵* is hydrogen and the other one is as defined above, in particular alkyl, more particularly methyl.

In a particular embodiment of the invention, R⁵ and R⁵* are independently selected from hydrogen, halogen, alkyl, alkoxyalkyl and azido, in particular from hydrogen, fluoro, methyl, methoxyethyl and azido. In particular, advantageous embodiments of the invention, one of R⁵ and R⁵* is hydrogen and the other one is alkyl, in particular methyl, halogen, in particular fluoro, alkoxyalkyl, in particular methoxyethyl or azido; or R⁵ and R⁵* are both hydrogen or halogen at the same time, in particular both hydrogen of fluoro at the same time. In such particular embodiments, W can advantageously be oxygen, and -X-Y- advantageously -O-CH₂-.

In a particular embodiment of the invention, -X-Y- is -O-CH₂-, W is oxygen and R¹, R², R³, R⁵ and R⁵* are all hydrogen at the same time. Such LNA nucleosides are disclosed in WO 99/014226, WO 00/66604, WO 98/039352 and WO 2004/046160 and include what are commonly known in the art as beta-D-oxy LNA and alpha-L-oxy LNA nucleosides.

In another particular embodiment of the invention, -X-Y- is -S-CH₂-, W is oxygen and R¹, R², R³, R⁵ and R^{5*} are all hydrogen at the same time. Such thio LNA nucleosides are disclosed in WO 99/014226 and WO 2004/046160.

In another particular embodiment of the invention, -X-Y- is -NH-CH₂-, W is oxygen and R¹, R², R³, R⁵ and R⁵* are all hydrogen at the same time. Such amino LNA nucleosides are disclosed in WO 99/014226 and WO 2004/046160.

In another particular embodiment of the invention, -X-Y- is -O-CH₂CH₂- or - OCH₂CH₂CH₂-, W is oxygen, and R¹, R², R³, R⁵ and R⁵* are all hydrogen at the same time. Such LNA nucleosides are disclosed in WO 00/047599 and Morita et al., Bioorganic & Med.Chem. Lett. 12, 73-76 and include what are commonly known in the art as 2'-O-4'C-ethylene bridged nucleic acids (ENA).

In another particular embodiment of the invention, -X-Y- is -O-CH₂-, W is oxygen, R¹, R², R³ are all hydrogen at the same time, one of R⁵ and R⁵* is hydrogen and the other one is not hydrogen, such as alkyl, for example methyl. Such 5' substituted LNA nucleosides are disclosed in WO 2007/134181.

In another particular embodiment of the invention, -X-Y- is -O-CR^{a}R^{b}-, wherein one or both of R^{a} and R^{b} are not hydrogen, in particular alkyl such as methyl, W is oxygen, R¹, R², R³ are all hydrogen at the same time, one of R⁵ and R⁵* is hydrogen and the other one is not hydrogen, in particular alkyl, for example methyl. Such bis modified LNA nucleosides are disclosed in WO 2010/077578.

In another particular embodiment of the invention, -X-Y- is -O-CHR^{a}-, W is oxygen and R¹, R², R³, R⁵ and R⁵* are all hydrogen at the same time. Such 6'-substituted LNA nucleosides are disclosed in WO 2010/036698 and WO 2007/. In such 6'-substituted LNA nucleosides, R^{a} is in particular C₁-C₆ alkyl, such as methyl.

In another particular embodiment of the invention, -X-Y- is -O-CH(CH₂-O-CH₃)-("2' O-methoxyethyl bicyclic nucleic acid", Seth et al. J. Org. Chem. 2010, Vol 75(5) pp. 1569-81).

In another particular embodiment of the invention, -X-Y- is -O-CH(CH₂CH₃)-;

In another particular embodiment of the invention, -X-Y- is -O-CH(CH₂-O-CH₃)-, W is oxygen and R¹, R², R³, R⁵ and R⁵* are all hydrogen at the same time. Such LNA nucleosides are also known in the art as cyclic MOEs (cMOE) and are disclosed in WO 2007/090071.

In another particular embodiment of the invention, -X-Y- is -O-CH(CH₃)- ("2'O-ethyl bicyclic nucleic acid", Seth at al., J. Org. Chem. 2010, Vol 75(5) pp. 1569-81).

In another particular embodiment of the invention, -X-Y- is -O-CH₂-O-CH₂- (Seth et al., J. Org. Chem 2010 op. cit.)
In another particular embodiment of the invention, -X-Y- is -O-CH(CH₃)-, W is oxygen and R¹, R², R³, R⁵ and R⁵* are all hydrogen at the same time. Such 6'-methyl LNA nucleosides are also known in the art as cET nucleosides, and may be either (S)-cET or (R)-cET diastereoisomers, as disclosed in WO 2007/090071 (beta-D) and WO 2010/036698 (alpha-L).

In another particular embodiment of the invention, -X-Y- is -O-CR^{a}R^{b}-, wherein neither R^{a} nor R^{b} is hydrogen, W is oxygen and R¹, R², R³, R⁵ and R⁵* are all hydrogen at the same time. In a particular embodiment, R^{a} and R^{b} are both alkyl at the same time, in particular both methyl at the same time. Such 6'-di-substituted LNA nucleosides are disclosed in WO 2009/006478.

In another particualr embodiment of the invention, -X-Y- is -S-CHR^{a}-, W is oxygen and R¹, R², R³, R⁵ and R⁵* are all hydrogen at the same time. Such 6'-substituted thio LNA nucleosides are disclosed in WO 2011/156202. In a particular embodiment of such 6'-substituted thio LNA, R^{a} is alkyl, in particular methyl.

In a particular embodiment of the invention, -X-Y- is -C(=CH₂)C(R^{a}R^{b})-, - C(=CHF)C(R^{a}R^{b})- or -C(=CF₂)C(R^{a}R^{b})-, W is oxygen and R¹, R², R³, R⁵ and R⁵* are all hydrogen at the same time. R^{a} and R^{b} are advantagesously independently selected from hydrogen, halogen, alkyl and alkoxyalkyl, in particular hydrogen, methyl, fluoro and methoxymethyl. R^{a} and R^{b} are in particular both hydrogen or methyl at the same time or one of R^{a} and R^{b} is hydrogen and the other one is methyl. Such vinyl carbo LNA nucleosides are disclosed in WO 2008/154401 and WO 2009/067647.

In a particular embodiment of the invention, -X-Y- is -N(OR^{a})-CH₂-, W is oxygen and R¹, R², R³, R⁵ and R⁵* are all hydrogen at the same time. In a particular embodiment, R^{a} is alkyl such as methyl. Such LNA nucleosides are also known as N substituted LNAs and are disclosed in WO 2008/150729.

In a particular embodiment of the invention, -X-Y- is -O-N(R^{a})-, -N(R^{a})-O-, -NR^{a}-CR^{a}R^{b}-CR^{a}R^{b}- or -NR^{a}-CR^{a}R^{b}-, W is oxygen and R¹, R², R³, R⁵ and R⁵* are all hydrogen at the same time. R^{a} and R^{b} are advantagesously independently selected from hydrogen, halogen, alkyl and alkoxyalkyl, in particular hydrogen, methyl, fluoro and methoxymethyl. In a particular embodiment, R^{a} is alkyl, such as methyl, R^{b} is hydrogen or methyl, in particular hydrogen. (Seth et al., J. Org. Chem 2010 op. cit.).

In a particular embodiment of the invention, -X-Y- is -O-N(CH₃)- (Seth et al., J. Org. Chem 2010 op. cit.).

In a particular embodiment of the invention, R⁵ and R⁵* are both hydrogen at the same time. In another particular embodiment of the invention, one of R⁵ and R⁵* is hydrogen and the other one is alkyl, such as methyl. In such embodiments, R¹, R² and R³ can be in particular hydrogen and -X-Y- can be in particular -O-CH₂- or -O-CHC(R^{a})₃-, such as -O-CH(CH₃)-.

In a particular embodiment of the invention, -X-Y- is -CR^{a}R^{b}-O-CR^{a}R^{b}-, such as - CH₂-O-CH₂-, W is oxygen and R¹, R², R³, R⁵ and R⁵* are all hydrogen at the same time. In such particular embodiments, R^{a} can be in particular alkyl such as methyl, R^{b} hydrogen or methyl, in particular hydrogen. Such LNA nucleosides are also known as conformationally restricted nucleotides (CRNs) and are disclosed in WO 2013/036868.

In a particular embodiment of the invention, -X-Y- is -O-CR^{a}R^{b}-O-CR^{a}R^{b}-, such as - O-CH₂-O-CH₂-, W is oxygen and R¹, R², R³, R⁵ and R⁵* are all hydrogen at the same time. R^{a} and R^{b} are advantagesously independently selected from hydrogen, halogen, alkyl and alkoxyalkyl, in particular hydrogen, methyl, fluoro and methoxymethyl. In such a particular embodiment, R^{a} can be in particular alkyl such as methyl, R^{b} hydrogen or methyl, in particular hydrogen. Such LNA nucleosides are also known as COC nucleotides and are disclosed in Mitsuoka et al., Nucleic Acids Research 2009, 37(4), 1225-1238,.

It will be recognized than, unless specified, the LNA nucleosides may be in the beta-D or alpha-L stereoisoform.

Particular examples of LNA nucleosides of the invention are presented in Scheme 1 (wherein B is as defined above).

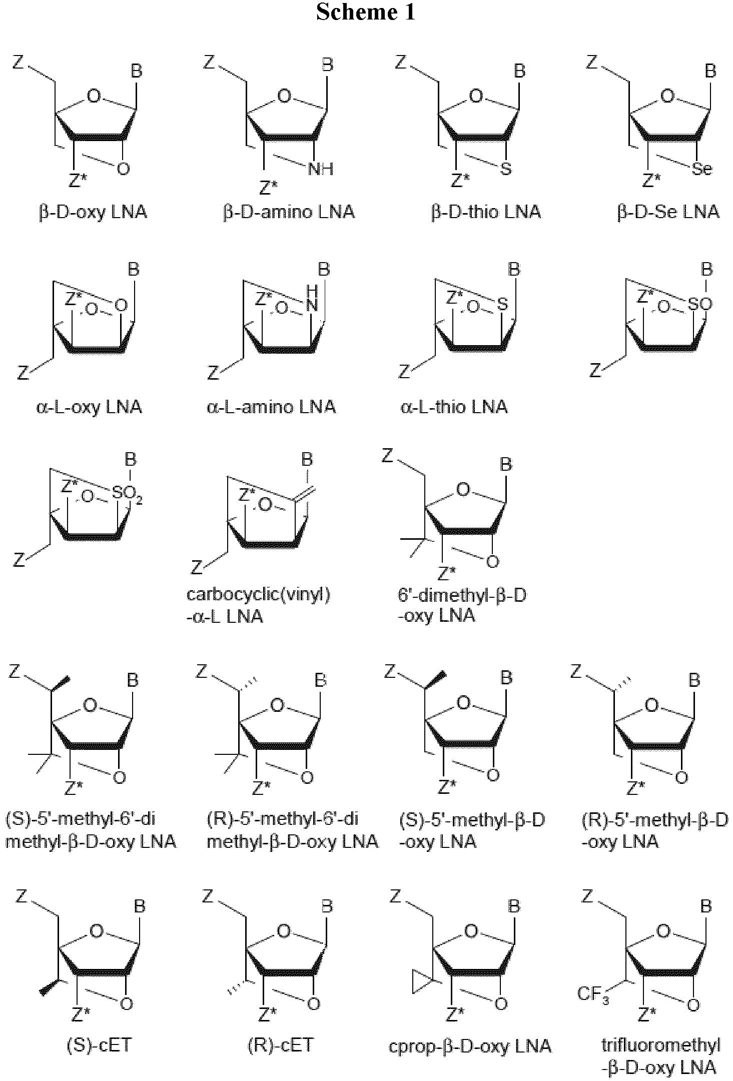

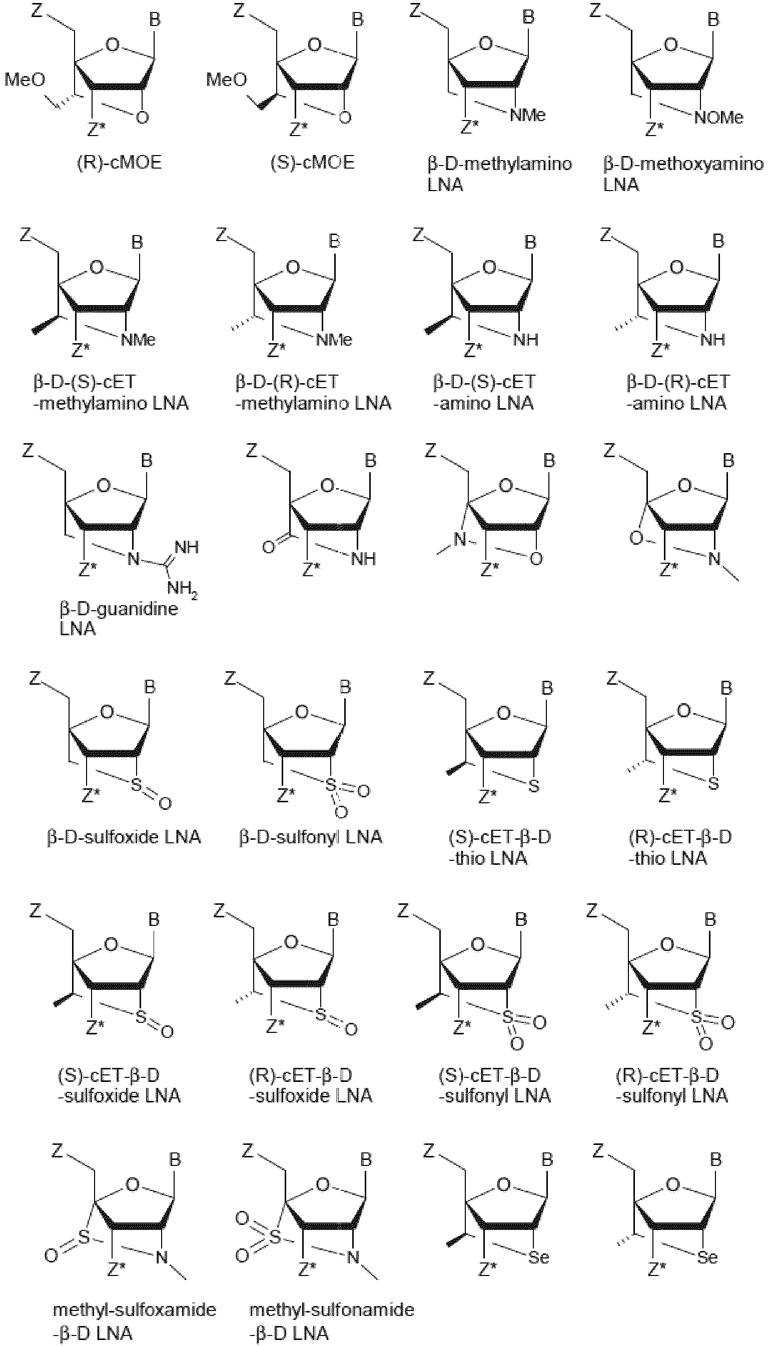

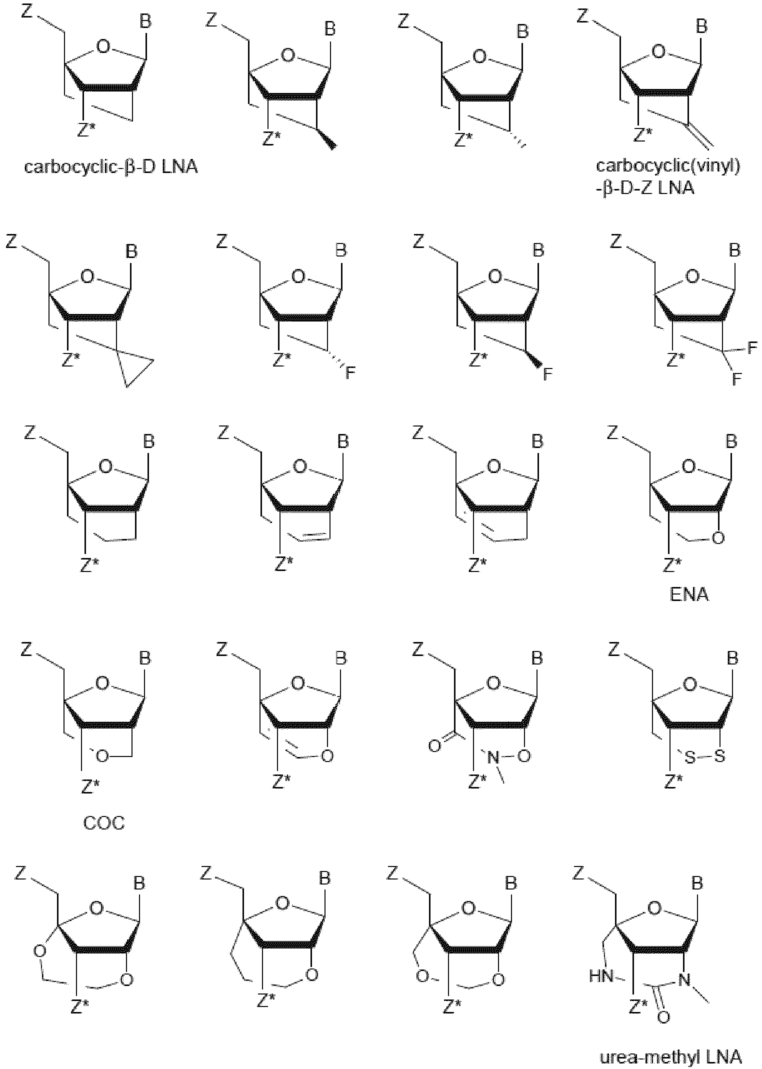

Particular LNA nucleosides are beta-D-oxy-LNA, 6'-methyl-beta-D-oxy LNA such as (S)-6'-methyl-beta-D-oxy-LNA ((S)-cET) and ENA.

### RNase H Activity and Recruitment

The RNase H activity of an antisense oligonucleotide refers to its ability to recruit RNase H when in a duplex with a complementary RNA molecule. WO01/23613 provides *in vitro* methods for determining RNaseH activity, which may be used to determine the ability to recruit RNaseH. Typically an oligonucleotide is deemed capable of recruiting RNase H if it, when provided with a complementary target nucleic acid sequence, has an initial rate, as measured in pmol/l/min, of at least 5%, such as at least 10% or more than 20% of the of the initial rate determined when using a oligonucleotide having the same base sequence as the modified oligonucleotide being tested, but containing only DNA monomers with phosphorothioate linkages between all monomers in the oligonucleotide, and using the methodology provided by Example 91 - 95 of WO01/23613. For use in determining RHase H activity, recombinant human RNase H1 is available from Lubio Science GmbH, Lucerne, Switzerland.

### Gapmer

The antisense oligonucleotide of the invention, or contiguous nucleotide sequence thereof may be a gapmer. The antisense gapmers are commonly used to inhibit a target nucleic acid via RNase H mediated degradation. A gapmer oligonucleotide comprises at least three distinct structural regions a 5'-flank, a gap and a 3'-flank, F-G-F' in the '5 -> 3' orientation. The "gap" region (G) comprises a stretch of contiguous DNA nucleotides which enable the oligonucleotide to recruit RNase H. The gap region is flanked by a 5' flanking region (F) comprising one or more sugar modified nucleosides, advantageously high affinity sugar modified nucleosides, and by a 3' flanking region (F') comprising one or more sugar modified nucleosides, advantageously high affinity sugar modified nucleosides. The one or more sugar modified nucleosides in region F and F' enhance the affinity of the oligonucleotide for the target nucleic acid (*i.e.* are affinity enhancing sugar modified nucleosides). In some embodiments, the one or more sugar modified nucleosides in region F and F' are 2' sugar modified nucleosides, such as high affinity 2' sugar modifications, such as independently selected from LNA and 2'-MOE.

In a gapmer design, the 5' and 3' most nucleosides of the gap region are DNA nucleosides, and are positioned adjacent to a sugar modified nucleoside of the 5' (F) or 3' (F') region respectively. The flanks may be further defined by having at least one sugar modified nucleoside at the end most distant from the gap region, i.e. at the 5' end of the 5' flank and at the 3' end of the 3' flank.

Regions F-G-F' form a contiguous nucleotide sequence. Antisense oligonucleotides of the invention, or the contiguous nucleotide sequence thereof, may comprise a gapmer region of formula F-G-F'.

The overall length of the gapmer design F-G-F' may be, for example 12 to 32 nucleosides, such as 13 to 24, such as 14 to 22 nucleosides, Such as from 14 to17, such as 16 to18 nucleosides.

By way of example, the gapmer oligonucleotide of the present invention can be represented by the following formulae:

F₁₋₈-G₅₋₁₆-F'₁₋₈,

such as

F₁₋₈-G₇₋₁₆-F'₂₋₈

with the proviso that the overall length of the gapmer regions F-G-F' is at least 12, such as at least 14 nucleotides in length.

Regions F, G and F' are further defined below and can be incorporated into the F-G-F' formula.

### Gapmer - Region G

Region G (gap region) of the gapmer is a region of nucleosides which enables the oligonucleotide to recruit RNaseH, such as human RNase H1, typically DNA nucleosides. RNaseH is a cellular enzyme which recognizes the duplex between DNA and RNA, and enzymatically cleaves the RNA molecule. Suitable gapmers may have a gap region (G) of at least 5 or 6 contiguous DNA nucleosides, such as 5 - 16 contiguous DNA nucleosides, such as 6 - 15 contiguous DNA nucleosides, such as 7-14 contiguous DNA nucleosides, such as 8 - 12 contiguous DNA nucleotides, such as 8 - 12 contiguous DNA nucleotides in length. The gap region G may, in some embodiments consist of 6, 7, 8, 9, 10, 11, 12, 13, 14, 15 or 16 contiguous DNA nucleosides. Cytosine (C) DNA in the gap region may in some instances be methylated, such residues are either annotated as 5-methyl-cytosine (^{me}C or with an e instead of a c). Methylation of Cytosine DNA in the gap is advantageous if cg dinucleotides are present in the gap to reduce potential toxicity, the modification does not have significant impact on efficacy of the oligonucleotides.

In some embodiments the gap region G may consist of 6, 7, 8, 9, 10, 11, 12, 13, 14, 15 or 16 contiguous phosphorothioate linked DNA nucleosides. In some embodiments, all internucleoside linkages in the gap are phosphorothioate linkages.

Whilst traditional gapmers have a DNA gap region, there are numerous examples of modified nucleosides which allow for RNaseH recruitment when they are used within the gap region. Modified nucleosides which have been reported as being capable of recruiting RNaseH when included within a gap region include, for example, alpha-L-LNA, C4' alkylated DNA (as described in PCT/EP2009/050349 and Vester et al., Bioorg. Med. Chem. Lett. 18 (2008) 2296 - 2300), arabinose derived nucleosides like ANA and 2'F-ANA (Mangos et al. 2003 J. AM. CHEM. SOC. 125, 654-661), UNA (unlocked nucleic acid) (as described in Fluiter et al., Mol. Biosyst., 2009, 10, 1039). UNA is unlocked nucleic acid, typically where the bond between C2 and C3 of the ribose has been removed, forming an unlocked "sugar" residue. The modified nucleosides used in such gapmers may be nucleosides which adopt a 2' endo (DNA like) structure when introduced into the gap region, *i.e.* modifications which allow for RNaseH recruitment). In some embodiments the DNA Gap region (G) described herein may optionally contain 1 to 3 sugar modified nucleosides which adopt a 2' endo (DNA like) structure when introduced into the gap region.

### Region G - "Gap-breaker"

Alternatively, there are numerous reports of the insertion of a modified nucleoside which confers a 3' endo conformation into the gap region of gapmers, whilst retaining some RNaseH activity. Such gapmers with a gap region comprising one or more 3'endo modified nucleosides are referred to as "gap-breaker" or "gap-disrupted" gapmers, see for example WO2013/022984. Gap-breaker oligonucleotides retain sufficient region of DNA nucleosides within the gap region to allow for RNaseH recruitment. The ability of gapbreaker oligonucleotide design to recruit RNaseH is typically sequence or even compound specific - see Rukov et al. 2015 Nucl. Acids Res. Vol. 43 pp. 8476-8487, which discloses "gapbreaker" oligonucleotides which recruit RNaseH which in some instances provide a more specific cleavage of the target RNA. Modified nucleosides used within the gap region of gap-breaker oligonucleotides may for example be modified nucleosides which confer a 3'endo confirmation, such 2' -O-methyl (OMe) or 2'-O-MOE (MOE) nucleosides, or beta-D LNA nucleosides (the bridge between C2' and C4' of the ribose sugar ring of a nucleoside is in the beta conformation), such as beta-D-oxy LNA or ScET nucleosides.

As with gapmers containing region G described above, the gap region of gap-breaker or gap-disrupted gapmers, have a DNA nucleoside at the 5' end of the gap (adjacent to the 3' nucleoside of region F), and a DNA nucleoside at the 3' end of the gap (adjacent to the 5' nucleoside of region F'). Gapmers which comprise a disrupted gap typically retain a region of at least 3 or 4 contiguous DNA nucleosides at either the 5' end or 3' end of the gap region.

Exemplary designs for gap-breaker oligonucleotides include

F₁₋₈-[D₃₋₄-E₁- D 3-4]-F'1-8

F₁₋₈- [D ₁₋₄-E₁- D 3-4]-F'1-8

F₁₋₈- [D ₃₋₄-E₁- D ₁₋₄]-F'₁₋₈

wherein region G is within the brackets [Dₙ-Eᵣ- Dₘ], D is a contiguous sequence of DNA nucleosides, E is a modified nucleoside (the gap-breaker or gap-disrupting nucleoside), and F and F' are the flanking regions as defined herein, and with the proviso that the overall length of the gapmer regions F-G-F' is at least 12, such as at least 14 nucleotides in length.

In some embodiments, region G of a gap disrupted gapmer comprises at least 6 DNA nucleosides, such as 6, 7, 8, 9, 10, 11, 12, 13, 14, 15 or 16 DNA nucleosides. As described above, the DNA nucleosides may be contiguous or may optionally be interspersed with one or more modified nucleosides, with the proviso that the gap region G is capable of mediating RNaseH recruitment.

### Gapmer - flanking regions, F and F'

Region F is positioned immediately adjacent to the 5' DNA nucleoside of region G. The 3' most nucleoside of region F is a sugar modified nucleoside, such as a high affinity sugar modified nucleoside, for example a 2' substituted nucleoside, such as a MOE nucleoside, or an LNA nucleoside.

Region F' is positioned immediately adjacent to the 3' DNA nucleoside of region G. The 5' most nucleoside of region F' is a sugar modified nucleoside, such as a high affinity sugar modified nucleoside, for example a 2' substituted nucleoside, such as a MOE nucleoside, or an LNA nucleoside.

Region F is 1-8 contiguous nucleotides in length, such as 2-6, such as 3-4 contiguous nucleotides in length. Advantageously the 5' most nucleoside of region F is a sugar modified nucleoside. In some embodiments the two 5' most nucleoside of region F are sugar modified nucleoside. In some embodiments the 5' most nucleoside of region F is an LNA nucleoside. In some embodiments the two 5' most nucleoside of region F are LNA nucleosides. In some embodiments the two 5' most nucleoside of region F are 2' substituted nucleoside nucleosides, such as two 3' MOE nucleosides. In some embodiments the 5' most nucleoside of region F is a 2' substituted nucleoside, such as a MOE nucleoside.

Region F' is 2-8 contiguous nucleotides in length, such as 3-6, such as 4-5 contiguous nucleotides in length. Advantageously, embodiments the 3' most nucleoside of region F' is a sugar modified nucleoside. In some embodiments the two 3' most nucleoside of region F' are sugar modified nucleoside. In some embodiments the two 3' most nucleoside of region F' are LNA nucleosides. In some embodiments the 3' most nucleoside of region F' is an LNA nucleoside. In some embodiments the two 3' most nucleoside of region F' are 2' substituted nucleoside nucleosides, such as two 3' MOE nucleosides. In some embodiments the 3' most nucleoside of region F' is a 2' substituted nucleoside, such as a MOE nucleoside.

It should be noted that when the length of region F or F' is one, it is advantageously an LNA nucleoside.

In some embodiments, region F and F' independently consists of or comprises a contiguous sequence of sugar modified nucleosides. In some embodiments, the sugar modified nucleosides of region F may be independently selected from 2'-O-alkyl-RNA units, 2'-O-methyl-RNA, 2'-amino-DNA units, 2'-fluoro-DNA units, 2'-alkoxy-RNA, MOE units, LNA units, arabino nucleic acid (ANA) units and 2'-fluoro-ANA units.

In some embodiments, region F and F' independently comprises both LNA and a 2' substituted modified nucleosides (mixed wing design).

In some embodiments, region F and F' consists of only one type of sugar modified nucleosides, such as only MOE or only beta-D-oxy LNA or only ScET. Such designs are also termed uniform flanks or uniform gapmer design.

In some embodiments, all the nucleosides of region F or F', or F and F' are LNA nucleosides, such as independently selected from beta-D-oxy LNA, ENA or ScET nucleosides. In some embodiments region F consists of 1-5, such as 2-4, such as 3-4 such as 1, 2, 3, 4 or 5 contiguous LNA nucleosides. In some embodiments, all the nucleosides of region F and F' are beta-D-oxy LNA nucleosides.

In some embodiments, all the nucleosides of region F or F', or F and F' are 2' substituted nucleosides, such as OMe or MOE nucleosides. In some embodiments region F consists of 1, 2, 3, 4, 5, 6, 7, or 8 contiguous OMe or MOE nucleosides. In some embodiments only one of the flanking regions can consist of 2' substituted nucleosides, such as OMe or MOE nucleosides. In some embodiments it is the 5' (F) flanking region that consists 2' substituted nucleosides, such as OMe or MOE nucleosides whereas the 3' (F') flanking region comprises at least one LNA nucleoside, such as beta-D-oxy LNA nucleosides or cET nucleosides. In some embodiments it is the 3' (F') flanking region that consists 2' substituted nucleosides, such as OMe or MOE nucleosides whereas the 5' (F) flanking region comprises at least one LNA nucleoside, such as beta-D-oxy LNA nucleosides or cET nucleosides.

In some embodiments, all the modified nucleosides of region F and F' are LNA nucleosides, such as independently selected from beta-D-oxy LNA, ENA or ScET nucleosides, wherein region F or F', or F and F' may optionally comprise DNA nucleosides (an alternating flank, see definition of these for more details). In some embodiments, all the modified nucleosides of region F and F' are beta-D-oxy LNA nucleosides, wherein region F or F', or F and F' may optionally comprise DNA nucleosides (an alternating flank, see definition of these for more details).

In some embodiments the 5' most and the 3' most nucleosides of region F and F' are LNA nucleosides, such as beta-D-oxy LNA nucleosides or ScET nucleosides.

In some embodiments, the internucleoside linkage between region F and region G is a phosphorothioate internucleoside linkage. In some embodiments, the internucleoside linkage between region F' and region G is a phosphorothioate internucleoside linkage. In some embodiments, the internucleoside linkages between the nucleosides of region F or F', F and F' are phosphorothioate internucleoside linkages.

Further gapmer designs are disclosed in WO 2004/046160, WO 2007/146511 and WO 2008/113832.

### LNA Gapmer

An LNA gapmer is a gapmer wherein either one or both of region F and F' comprises or consists of LNA nucleosides. A beta-D-oxy gapmer is a gapmer wherein either one or both of region F and F' comprises or consists of beta-D-oxy LNA nucleosides.

In some embodiments the LNA gapmer is of formula: [LNA]₁₋₅-[region G] -[LNA]₁₋₅, wherein region G is as defined in the Gapmer region G definition.

### MOE Gapmers

A MOE gapmers is a gapmer wherein regions F and F' consist of MOE nucleosides. In some embodiments the MOE gapmer is of design [MOE]₁₋₈-[Region G]-[MOE] ₁₋₈, such as [MOE]₂₋₇-[Region G]₅₋₁₆-[MOE] ₂₋₇, such as [MOE]₃₋₆-[Region G]-[MOE] ₃₋₆, wherein region G is as defined in the Gapmer definition. MOE gapmers with a 5-10-5 design (MOE-DNA-MOE) have been widely used in the art.

### Mixed Wing Gapmer

A mixed wing gapmer is an LNA gapmer wherein one or both of region F and F' comprise a 2' substituted nucleoside, such as a 2' substituted nucleoside independently selected from the group consisting of 2'-O-alkyl-RNA units, 2'-O-methyl-RNA, 2'-amino-DNA units, 2'-fluoro-DNA units, 2'-alkoxy-RNA, MOE units, arabino nucleic acid (ANA) units and 2'-fluoro-ANA units, such as a MOE nucleoside. In some embodiments wherein at least one of region F and F', or both region F and F' comprise at least one LNA nucleoside, the remaining nucleosides of region F and F' are independently selected from the group consisting of MOE and LNA. In some embodiments wherein at least one of region F and F', or both region F and F' comprise at least two LNA nucleosides, the remaining nucleosides of region F and F' are independently selected from the group consisting of MOE and LNA. In some mixed wing embodiments, one or both of region F and F' may further comprise one or more DNA nucleosides.

Mixed wing gapmer designs are disclosed in WO 2008/049085 and WO 2012/109395.

### Alternating Flank Gapmers

Flanking regions may comprise both LNA and DNA nucleoside and are referred to as "alternating flanks" as they comprise an alternating motif of LNA-DNA-LNA nucleosides. Gapmers comprising such alternating flanks are referred to as "alternating flank gapmers". "Alternative flank gapmers" are thus LNA gapmer oligonucleotides where at least one of the flanks (F or F') comprises DNA in addition to the LNA nucleoside(s). In some embodiments at least one of region F or F', or both region F and F', comprise both LNA nucleosides and DNA nucleosides. In such embodiments, the flanking region F or F', or both F and F' comprise at least three nucleosides, wherein the 5' and 3' most nucleosides of the F and/or F' region are LNA nucleosides.

Alternating flank LNA gapmers are disclosed in WO 2016/127002.

An alternating flank region may comprise up to 3 contiguous DNA nucleosides, such as 1 to 2 or 1 or 2 or 3 contiguous DNA nucleosides.

The alternating flak can be annotated as a series of integers, representing a number of LNA nucleosides (L) followed by a number of DNA nucleosides (D), for example

[L]₁₋₃-[D]₁₋₄-[L]₁₋₃

[L]₁₋₂-[D]₁₋₂-[L]₁₋₂-[D]₁₋₂-[L]₁₋₂

In oligonucleotide designs these will often be represented as numbers such that 2-2-1 represents 5' [L]₂-[D]₂-[L] 3', and 1-1-1-1-1 represents 5' [L]-[D]-[L]-[D]-[L] 3'. The length of the flank (region F and F') in oligonucleotides with alternating flanks may independently be 3 to 10 nucleosides, such as 4 to 8, such as 5 to 6 nucleosides, such as 4, 5, 6 or 7 modified nucleosides. In some embodiments only one of the flanks in the gapmer oligonucleotide is alternating while the other is constituted of LNA nucleotides. It may be advantageous to have at least two LNA nucleosides at the 3' end of the 3' flank (F'), to confer additional exonuclease resistance. Some examples of oligonucleotides with alternating flanks are:

[L]₁₋₅-[D]₁₋₄-[L]₁₋₃-[G]₅₋₁₆-[L]₂₋₆

[L]₁₋₂-[D]₁₋₂-[L]₁₋₂-[D]₁₋₂-[L]₁₋₂-[G]₅₋₁₆-[L]₁₋₂-[D]₁₋₃-[L]₂₋₄

[L]₁₋₅-[G]₅₋₁₆-[L]-[D]-[L]-[D]-[L]₂

with the proviso that the overall length of the gapmer is at least 12, such as at least 14 nucleotides in length.

### Region D' or D" in an oligonucleotide

The oligonucleotide of the invention may in some embodiments comprise or consist of the contiguous nucleotide sequence of the oligonucleotide which is complementary to the target nucleic acid, such as the gapmer F-G-F', and further 5' and/or 3' nucleosides. The further 5' and/or 3' nucleosides may or may not be fully complementary to the target nucleic acid. Such further 5' and/or 3' nucleosides may be referred to as region D' and D'' herein.

The addition of region D' or D'' may be used for the purpose of joining the contiguous nucleotide sequence, such as the gapmer, to a conjugate moiety or another functional group. When used for joining the contiguous nucleotide sequence with a conjugate moiety is can serve as a biocleavable linker. Alternatively, it may be used to provide exonucleoase protection or for ease of synthesis or manufacture.

Region D' and D'' can be attached to the 5' end of region F or the 3' end of region F', respectively to generate designs of the following formulas D'-F-G-F', F-G-F'-D'' or
D'-F-G-F'-D''. In this instance the F-G-F' is the gapmer portion of the oligonucleotide and region D' or D'' constitute a separate part of the oligonucleotide.

Region D' or D'' may independently comprise or consist of 1, 2, 3, 4 or 5 additional nucleotides, which may be complementary or non-complementary to the target nucleic acid. The nucleotide adjacent to the F or F' region is not a sugar-modified nucleotide, such as a DNA or RNA or base modified versions of these. The D' or D' region may serve as a nuclease susceptible biocleavable linker (see definition of linkers). In some embodiments the additional 5' and/or 3' end nucleotides are linked with phosphodiester linkages, and are DNA or RNA. Nucleotide based biocleavable linkers suitable for use as region D' or D'' are disclosed in WO 2014/076195, which include by way of example a phosphodiester linked DNA dinucleotide. The use of biocleavable linkers in poly-oligonucleotide constructs is disclosed in WO 2015/113922, where they are used to link multiple antisense constructs (e.g. gapmer regions) within a single oligonucleotide.

In one embodiment the oligonucleotide of the invention comprises a region D' and/or D'' in addition to the contiguous nucleotide sequence which constitutes the gapmer.

In some embodiments, the oligonucleotide of the present invention can be represented by the following formulae:
F-G-F', in particular F₁₋₈-G₅₋₁₆-F'₂₋₈
D'-F-G-F', in particular D' ₁₋₃-F₁₋₈-G₅₋₁₆-F'₂₋₈
F-G-F'-D'', in particular F₁₋₈-G₅₋₁₆-F'₂₋₈-D''₁₋₃
D'-F-G-F'-D'', in particular D'₁₋₃- F₁₋₈-G₅₋₁₆-F'₂₋₈-D''₁₋₃

In some embodiments the internucleoside linkage positioned between region D' and region F is a phosphodiester linkage. In some embodiments the internucleoside linkage positioned between region F' and region D'' is a phosphodiester linkage.

### Totalmers

In some embodiments, all of the nucleosides of the oligonucleotide, or contiguous nucleotide sequence thereof, are sugar modified nucleosides. Such oligonucleotides are referred to as a totalmers herein.

In some embodiments all of the sugar modified nucleosides of a totalmer comprise the same sugar modification, for example they may all be LNA nucleosides, or may all be 2'O-MOE nucleosides. In some embodiments the sugar modified nucleosides of a totalmer may be independently selected from LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments the oligonucleotide comprises both LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments, the oligonucleotide comprises LNA nucleosides and 2'-O-MOE nucleosides. In some embodiments, the oligonucleotide comprises (S)cET LNA nucleosides and 2'-O-MOE nucleosides. In some embodiments, each nucleoside unit of the oligonucleotide is a 2'substituted nucleoside. In some embodiments, each nucleoside unit of the oligonucleotide is a 2'-O-MOE nucleoside.

In some embodiments, all of the nucleosides of the oligonucleotide or contiguous nucleotide sequence thereof are LNA nucleosides, such as beta-D-oxy-LNA nucleosides and/or (S)cET nucleosides. In some embodiments such LNA totalmer oligonucleotides are between 7 - 12 nucleosides in length (see for example, WO 2009/043353). Such short fully LNA oligonucelotides are particularly effective in inhibiting microRNAs.

Various totalmer compounds are highly effective as therapeutic oligomers, particularly when targeting microRNA (antimiRs) or as splice switching oligomers (SSOs).

In some embodiments, the totalmer comprises or consists of at least one XYX or YXY sequence motif, such as a repeated sequence XYX or YXY, wherein X is LNA and Y is an alternative (i.e. non LNA) nucleotide analogue, such as a 2'-OMe RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 7 and 24 nucleotides, such as 7, 9, 10, 11, 12, 13, 14, 15, 16, 17, 18, 19, 20, 21, 22 or 23 nucleotides.

In some embodiments, the contiguous nucleotide sequence of the totolmer comprises of at least 30%, such as at least 40%, such as at least 50%, such as at least 60%, such as at least 70%, such as at least 80%, such as at least 90%, such as 95%, such as 100% LNA units. For full LNA compounds, it is advantageous that they are less than 12 nucleotides in length, such as 7 - 10.

The remaining units may be selected from the non-LNA nucleotide analogues referred to herein in, such those selected from the group consisting of 2'-O-alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group 2'-OMe RNA unit and 2'-fluoro DNA unit.

### Mixmers

The term 'mixmer' refers to oligomers which comprise both DNA nucleosides and sugar modified nucleosides, wherein there are insufficient length of contiguous DNA nucleosides to recruit RNaseH. Suitable mixmers may comprise up to 3 or up to 4 contiguous DNA nucleosides. In some embodiments the mixmers comprise alternating regions of sugar modified nucleosides, and DNA nucleosides. By alternating regions of sugar modified nucleosides which form a RNA like (3'endo) conformation when incorporated into the oligonucleotide, with short regions of DNA nucleosides, non-RNaseH recruiting oligonucleotides may be made. Advantageously, the sugar modified nucleosides are affinity enhancing sugar modified nucleosides.

Oligonucleotide mixmers are often used to provide occupation based modulation of target genes, such as splice modulators or microRNA inhibitors.

In some embodiments the sugar modified nucleosides in the mixmer, or contiguous nucleotide sequence thereof, comprise or are all LNA nucleosides, such as (S)cET or beta-D-oxy LNA nucleosides.

In some embodiments all of the sugar modified nucleosides of a mixmer comprise the same sugar modification, for example they may all be LNA nucleosides, or may all be 2'O-MOE nucleosides. In some embodiments the sugar modified nucleosides of a mixmer may be independently selected from LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments the oligonucleotide comprises both LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments, the oligonucleoitide comprises LNA nucleosides and 2'-O-MOE nucleosides. In some embodiments, the oligonucleotide comprises (S)cET LNA nucleosides and 2'-O-MOE nucleosides.

In some embodiments the mixmer, or continguous nucleotide sequence thereof, comprises only LNA and DNA nucleosides, such LNA mixmer oligonucleotides which may for example be between 8 - 24 nucleosides in length (see for example, WO2007112754, which discloses LNA antmiR inhibitors of microRNAs).

Various mixmer compounds are highly effective as therapeutic oligomers, particularly when targeting microRNA (antimiRs) or as splice switching oligomers (SSOs).

In some embodiments, the mixmer comprises a motif

...[L]m[D]n[L]m[D]n[L]m...

or

...[L]m[D]n[L]m[D]n[L]m[D]n[L]m ...

or

...[L]m[D]n[L]m[D]n[L]m[D]n[L]m[D]n[L]m ...

or

...[L]m[D]n[L]m[D]n[L]m[D]n[L]m[D]n[L]m[D]n[L]m ...

Wherein L represents sugar modified nucleoside such as a LNA or 2' substituted nucleoside (e.g. 2'-O-MOE), D represents DNA nucleoside, and wherein each m is independently selected from 1 - 6, and each n is independently selected from 1, 2, 3 and 4, such as 1- 3. In some embodiments each L is a LNA nucleoside. In some embodiments, at least one L is a LNA nucleoside and at least one L is a 2'-O-MOE nucleoside. In some embodiments, each L is independently selected from LNA and 2'-O-MOE nucleoside.

In some embodiments, the mixmer may comprise or consist of a contiguous nucleotide sequence of between 10 and 24 nucleotides, such as 11, 12, 13, 14, 15, 16, 17, 18, 19, 20, 21, 22 or 23 nucleotides.

In some embodiments, the contiguous nucleotide sequence of the mixmer comprises of at least 30%, such as at least 40%, such as at least 50% LNA units.

In some embodiments, the mixmer comprises or consists of a contiguous nucleotide sequence of repeating pattern of nucleotide analogues and naturally occurring nucleotides, or one type of nucleotide analogue and a second type of nucleotide analogue. The repeating pattern, may, for instance be: every second or every third nucleotide is a nucleotide analogue, such as LNA, and the remaining nucleotides are naturally occurring nucleotides, such as DNA, or are a 2' substituted nucleotide analogue such as 2'MOE of 2'fluoro analogues as referred to herein, or, in some embodiments selected form the groups of nucleotide analogues referred to herein. It is recognised that the repeating pattern of nucleotide analogues, such as LNA units, may be combined with nucleotide analogues at fixed positions - e.g. at the 5' or 3' termini.

In some embodiments the first nucleotide of the oligomer, counting from the 3' end, is a nucleotide analogue, such as a LNA nucleotide or a 2'-O-MOE nucleoside.

In some embodiments, which maybe the same or different, the second nucleotide of the oligomer, counting from the 3' end, is a nucleotide analogue, such as a LNA nucleotide or a 2'-O-MOE nucleoside.

In some embodiments, which maybe the same or different, the 5' terminal of the oligomer is a nucleotide analogue, such as a LNA nucleotide or a 2'-O-MOE nucleoside.

In some embodiments, the mixmer comprises at least a region comprising at least two consecutive nucleotide analogue units, such as at least two consecutive LNA units.

In some embodiments, the mixmer comprises at least a region comprising at least three consecutive nucleotide analogue units, such as at least three consecutive LNA units.

### Conjugate

The term conjugate as used herein refers to an oligonucleotide which is covalently linked to a non-nucleotide moiety (conjugate moiety or region C or third region).

Conjugation of the oligonucleotide of the invention to one or more non-nucleotide moieties may improve the pharmacology of the oligonucleotide, e.g. by affecting the activity, cellular distribution, cellular uptake or stability of the oligonucleotide. In some embodiments the conjugate moiety modifies or enhances the pharmacokinetic properties of the oligonucleotide by improving cellular distribution, bioavailability, metabolism, excretion, permeability, and/or cellular uptake of the oligonucleotide. In particular, the conjugate may target the oligonucleotide to a specific organ, tissue or cell type and thereby enhance the effectiveness of the oligonucleotide in that organ, tissue or cell type. At the same time the conjugate may serve to reduce activity of the oligonucleotide in non-target cell types, tissues or organs, e.g. off target activity or activity in non-target cell types, tissues or organs.

WO 93/07883 and WO 2013/033230 provides suitable conjugate moieties. Further suitable conjugate moieties are those capable of binding to the asialoglycoprotein receptor (ASGPR). In particular tri-valent N-acetylgalactosamine conjugate moieties are suitable for binding to the ASGPR, see for example WO 2014/076196, WO 2014/207232 and WO 2014/179620. Such conjugates serve to enhance uptake of the oligonucleotide to the liver while reducing its presence in the kidney, thereby increasing the liver/kidney ratio of a conjugated oligonucleotide compared to the unconjugated version of the same oligonucleotide.

Oligonucleotide conjugates and their synthesis has also been reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103.

In an embodiment, the non-nucleotide moiety (conjugate moiety) is selected from the group consisting of carbohydrates, cell surface receptor ligands, drug substances, hormones, lipophilic substances, polymers, proteins, peptides, toxins (e.g. bacterial toxins), vitamins, viral proteins (e.g. capsids) or combinations thereof.

### Linkers

A linkage or linker is a connection between two atoms that links one chemical group or segment of interest to another chemical group or segment of interest via one or more covalent bonds. Conjugate moieties can be attached to the oligonucleotide directly or
through a linking moiety (e.g. linker or tether). Linkers serve to covalently connect a third
region, e.g. a conjugate moiety (Region C), to a first region, e.g. an oligonucleotide or contiguous nucleotide sequence complementary to the target nucleic acid (region A).

In some embodiments of the invention the conjugate or oligonucleotide conjugate of the invention may optionally, comprise a linker region (second region or region B and/or region Y) which is positioned between the oligonucleotide or contiguous nucleotide sequence complementary to the target nucleic acid (region A or first region) and the conjugate moiety (region C or third region).

Region B refers to biocleavable linkers comprising or consisting of a physiologically labile bond that is cleavable under conditions normally encountered or analogous to those encountered within a mammalian body. Conditions under which physiologically labile linkers undergo chemical transformation (e.g., cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. In one embodiment the biocleavable linker is susceptible to S1 nuclease cleavage. In a preferred embodiment the nuclease susceptible linker comprises between 1 and 10 nucleosides, such as 1, 2, 3, 4, 5, 6, 7, 8, 9 or 10 nucleosides, more preferably between 2 and 6 nucleosides and most preferably between 2 and 4 linked nucleosides comprising at least two consecutive phosphodiester linkages, such as at least 3 or 4 or 5 consecutive phosphodiester linkages. Preferably the nucleosides are DNA or RNA. Phosphodiester containing biocleavable linkers are described in more detail in WO 2014/076195.

Region Y refers to linkers that are not necessarily biocleavable but primarily serve to covalently connect a conjugate moiety (region C or third region), to an oligonucleotide (region A or first region). The region Y linkers may comprise a chain structure or an oligomer of repeating units such as ethylene glycol, amino acid units or amino alkyl groups The oligonucleotide conjugates of the present invention can be constructed of the following regional elements A-C, A-B-C, A-B-Y-C, A-Y-B-C or A-Y-C. In some embodiments the linker (region Y) is an amino alkyl, such as a C2 - C36 amino alkyl group, including, for example C6 to C12 amino alkyl groups. In a preferred embodiment the linker (region Y) is a C6 amino alkyl group.

The oligonucleotide according to the invention is thus of formula (II) 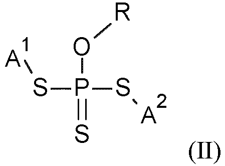 wherein (A¹) and (A²) are as defined above.

The invention thus relates in particular to:
An oligonucleotide according to the invention wherein the nucleoside (A¹) is a DNA nucleoside;
An oligonucleotide according to the invention wherein the nucleoside (A²) is a DNA nucleoside, a RNA nucleoside or a sugar modified nucleoside;
An oligonucleotide according to the invention wherein the nucleoside (A²) is a DNA nucleoside or a sugar modified nucleoside;
An oligonucleotide according to the invention wherein the sugar modified nucleoside is a 2' sugar modified nucleoside;
An oligonucleotide according to the invention wherein the nucleoside (A²) is 2'-alkoxy-RNA, in particular 2'-methoxy-RNA, 2'-alkoxyalkoxy-RNA, in particular 2'-methoxyethoxy-RNA, 2'-amino-DNA, 2'-fluoro-RNA or 2'-fluoro-ANA;
An oligonucleotide according to the invention wherein the nucleoside (A²) is a LNA nucleoside;
An oligonucleotide according to the invention wherein the LNA nucleoside is independently selected from beta-D-oxy LNA, 6'-methyl-beta-D-oxy LNA and ENA, in particular beta-D-oxy LNA;
An oligonucleotide according to the invention wherein at least one of the nucleosides (A¹) and (A²) is a 2'-alkoxyalkoxy-RNA;
An oligonucleotide according to the invention wherein the 2'-alkoxyalkoxy-RNA is 2'-methoxyethoxy-RNA;
An oligonucleotide according to the invention wherein the nucleosides (A¹) and (A²) are both DNA nucleosides;
An oligonucleotide according to the invention comprising further internucleoside linkages selected from phosphodiester internucleoside linkage, phosphorothioate internucleoside linkage and phosphorotrithioate internucleoside linkage of formula (I) as defined above;
An oligonucleotide according to the invention comprising further internucleoside linkages selected from phosphorothioate internucleoside linkage and phosphorotrithioate internucleoside linkage of formula (I) as defined above;
An oligonucleotide according to the invention comprising between 1 and 15, in particular between 1 and 5, more particularly 1, 2, 3, 4 or 5 phosphorotrithioate internucleoside linkages of formula (I) as defined above;
An oligonucleotide according to the invention wherein the further internucleoside linkages are all phosphorothioate internucleoside linkages of formula -P(=S)(OR)O₂-, wherein R is as defined above;
An oligonucleotide according to the invention comprising further nucleosides selected from DNA nucleoside, RNA nucleoside and sugar modified nucleosides;
An oligonucleotide according to the invention wherein one or more nucleoside is a nucleobase modified nucleoside;
An oligonucleotide according to the invention wherein the oligonucleotide is an antisense oligonucleotide, an siRNA, a microRNA mimic or a ribozyme;
An oligonucleotide according to the invention wherein the oligonucleotide is an antisense gapmer oligonucleotide;
An oligonucleotide according to the invention, wherein the phosphorotrithioate internucleoside linkage of formula (I) is in the gap region of the gapmer oligonucleotide;
An oligonucleotide according to the invention wherein the gapmer oligonucleotide is a LNA gapmer, a mixed wing gapmer or a 2'-substituted gapmer, in particular a 2'-O-methoxyethyl gapmer;
A gapmer oligonucleotide according to the invention wherein the gapmer oligonucleotide comprises a contiguous nucleotide sequence of formula 5'-F-G-F'-3', wherein G is a region of 5 to18 nucleosides which is capable of recruiting RnaseH, and said region G is flanked 5' and 3' by flanking regions F and F' respectively, wherein regions F and F' independently comprise or consist of 1 to 7 2'-sugar modified nucleotides, wherein the nucleoside of region F which is adjacent to region G is a 2'-sugar modified nucleoside and wherein the nucleoside of region F' which is adjacent to region G is a 2'-sugar modified nucleoside;
A gapmer oligonucleotide according to the invention wherein said at least one phosphorotrithioate internucleoside linkage of formula (I) as defined above is positioned between adjacent nucleosides in region G or between region G and region F';
An oligonucleotide according to the invention wherein the oligonucleotide is an antisense oligonucleotide mixmer or totalmer, in particular a splice-switching oligonucleotide or a microRNA inhibitor oligonucleotide;
A pharmaceutically acceptable salt of an oligonucleotide according to the invention, in particular a sodium or a potassium salt;
A conjugate comprising an oligonucleotide or a pharmaceutically acceptable salt according to the invention and at least one conjugate moiety covalently attached to said oligonucleotide or said pharmaceutically acceptable salt, optionally *via* a linker moiety;
A pharmaceutical composition comprising an oligonucleotide, a pharmaceutically acceptable salt or a conjugate according to the invention and a therapeutically inert carrier;
An oligonucleotide, pharmaceutically acceptable salt or conjugate according to the invention for use as therapeutically active substance;
An oligonucleotide, pharmaceutically acceptable salt or conjugate according to the invention for use in the treatment or prophylaxis of a heart or blood disease;
The use of an oligonucleotide, pharmaceutically acceptable salt or conjugate according to the invention for the preparation of a medicament for the treatment or prophylaxis of a heart or blood disease;
The use of an oligonucleotide, pharmaceutically acceptable salt or conjugate according to the invention in the treatment or prophylaxis of a heart or blood disease;
A method for the treatment or prophylaxis of a heart or blood disease comprising the administration of an effective amount of an oligonucleotide, pharmaceutically acceptable salt or conjugate according to the invention to a patient in need thereof;
A method of inhibiting a target RNA, in particular a human mRNA or a viral RNA, in a cell comprising administering an oligonucleotide according to the invention to a cell expressing said target RNA;
An in vitro method of modulating or inhibiting a target RNA, in particular a human mRNA or a viral RNA, in a cell comprising administering an oligonucleotide or gapmer oligonucleotide according to the invention to a cell expressing said target RNA;
A process for the manufacture of an oligonucleotide according to the invention comprising the following steps:
   (a) Coupling a 3'S-modified nucleoside phosphoramidite to the terminal 5' sulfur atom of a 5'S- modified nucleoside or oligonucleotide to produce a dithiophosphite triester intermediate;
   (b) Thiooxidizing the dithiophosphite triester intermediate obtained in step a); and
   (c) Optionally further elongating the oligonucleotide;
A process according to the invention wherein the 5' S-modified nucleoside or oligonulceotide of step (a) is attached to a solid support;
A process according to the invention further comprising the cleavage of the oligonucleotide from the solid support; and
An oligonucleotide manufactured according to a process of the invention.

In some embodiments, the oligonucleotide of the invention has a higher activity in modulating its target nucleic acid, as compared to the corresponding fully phosphorothioate linked-oligonucleotide. In some embodiments the invention provides for oligonucleotides with enhanced activity, enhanced potency, enhanced specific activity or enhanced cellular uptake. In some embodiments the invention provides for oligonucleotides which have an altered duration of action *in vitro* or *in vivo,* such as a prolonged duration of action *in vitro* or *in vivo.* In some embodiments the higher activity in modulating the target nucleic acid is determined *in vitro* or *in vivo* in a cell which is expressing the target nucleic acid.

In some embodiments the oligonucleotide of the invention has altered pharmacological properties, such as reduced toxicity, for example reduced nephrotoxicity, reduced hepatotoxicity or reduced immune stimulation. Hepatotoxicity may be determined, for example *in vivo,* or by using the in vitro assays disclosed in WO 2017/067970. Nephrotoxicity may be determined, for example *in vitro,* or by using the assays disclosed in PCT/EP2017/064770. In some embodiments the oligonucleotide of the invention comprises a 5' CG 3' dinucleotide, such as a DNA 5' CG 3' dinucleotide,
wherein the internucleoside linkage between C and G is a phosphorotrithioate internucleoside linkage of formula (I) as defined above.

In some embodiments, the oligonucleotide of the invention has improved nuclease resistance such as improved biostability in blood serum. In some embodiments, the 3' terminal nucleoside of the oligonucleotide of the invention has an A or G base, such as a 3' terminal LNA-A or LNA-G nucleoside. Suitably, the internucleoside linkage between the two 3' most nucleosides of the oligonucleotide may be a phosphorotrithioate internucleoside linkage according to formula (I) as defined above.

In some embodiments the oligonucleotide of the invention has enhanced bioavailability. In some embodiments the oligonucleotide of the invention has a greater blood exposure, such as a longer retention time in blood.

The oligonucleotide according to the invention can for example be prepared according to the following schemes.

Trithiophosphate linkages bearing sulfur atoms in the nonbridging position as well as in 3' and 5' positions of the adjacent furanose rings can be introduced into oligonucleotides by solid phase synthesis with the phosphoramidite method. Syntheses are performed using controlled pore glass (CPG) equipped with an universal linker as the support. On such a solid support an oligonucleotide is typically built up in a 3' to 5' direction by means of sequencial cycles consisting of coupling of 5'O-DMT protected nucleoside phosphoramidite building blocks followed by (thio)oxydation, capping and deprotection of the DMT group. For the introduction of a phosphorotrithioate as described in this application a DMT protected 5'-deoxy-5'-mercapto phosphoramidite building block is coupled to the free 5'-hydroxy group of a solid support bound oligonucleotide chain. After (thio)oxydation and capping the resulting intermediate is then deprotected to free a 5'-thiol group.

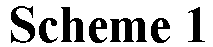

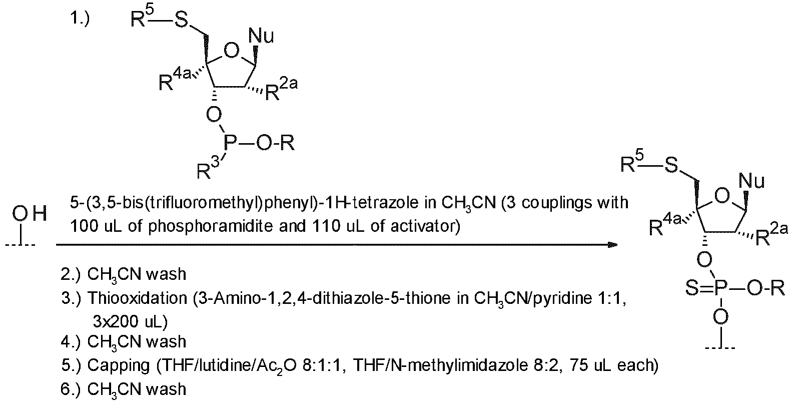

This deprotection is challenging and best performed either by repeated (more than 15 times) application of standard deprotection conditions (typically 3-5% of dichloroacetic acid or trichloroacetic acid in dichloromethane) or using higher acid concentrations (e.g. up to 10% trichloroacetic acid in dichloromethane) or stronger acids (e.g. 5% trifluoroacetic acid in dichloromethane) preferencially using appropriate cation scavengers (typically 20% triethylsilane, 5% 4-Methoxythiophenol or a combination of both).

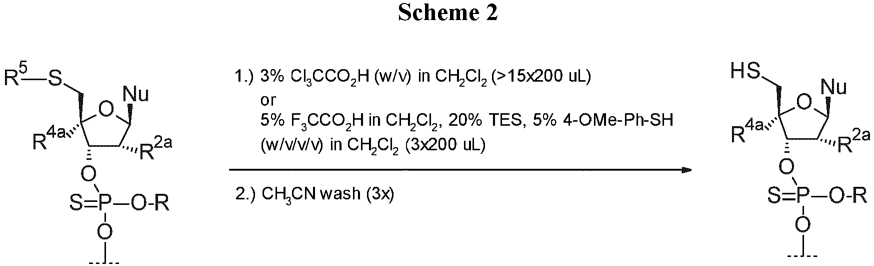

The free 5' thiol group thus obtained is then coupled to an appropriate 5'O-DMT protected 3'-deoxy-3'-mercapto phosphoramidite building block. Due to the lower reactivity of such building blocks typical procedures include 12 couplings using 4 equivalents and an extended reaction time (7.5 min). The resulting dithiophosphite intermediate can then undergo thiooxydation using an appropriate reagent (e.g. 3-Amino-1,2,4-dithiazole-5-thione) to provide the desired phorphorotrithioate linkage.

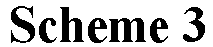

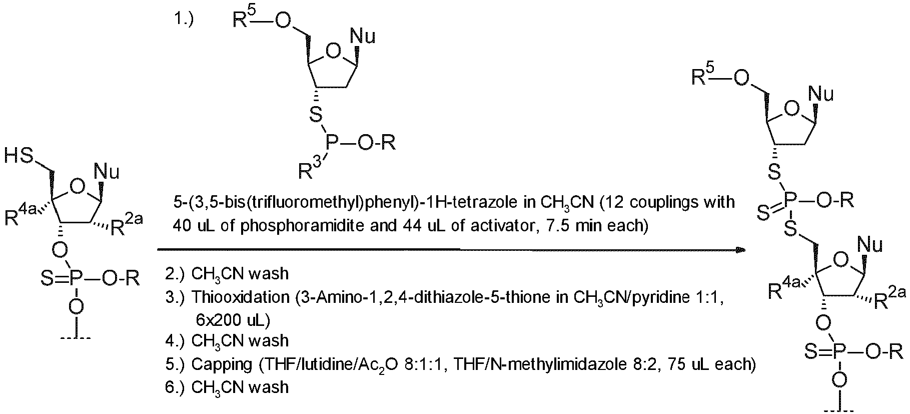

In the above schemes:
R^{2a} and R^{4a} together form -X-Y- as defined above; or
R^{4a} is hydrogen and R^{2a} is selected from alkoxy, in particular methoxy, halogen, in particular fluoro, alkoxyalkoxy, in particular methoxyethoxy, alkenyloxy, in particular allyloxy and aminoalkoxy, in particular aminoethyloxy;
R^{2b} and R^{4b} together form -X-Y- as defined above; or
R^{2b} and R^{4b} are both hydrogen at the same time; or
R^{4b} is hydrogen and R^{2b} is selected from alkoxy, in particular methoxy, halogen, in particular fluoro, alkoxyalkoxy, in particular methoxyethoxy, alkenyloxy, in particular allyloxy and aminoalkoxy, in particular aminoethyloxy;
R³ is dialkylamino or pyrrolidinyl;
R⁵ is a hydroxyl or thiohydroxyl protecting group; and
R is as defined above.

The invention will now be illustrated by the following examples which have no limiting character.

### Examples

### Example 1

### Oligonucleotide synthesis

Oligonucleotides were synthesized using a MerMade 12 automated DNA synthesizer by Bioautomation. Syntheses were conducted on a 1 µmol scale using a controlled pore glass support (500Å) bearing a universal linker.

In standard cycle procedures for the coupling of DNA and LNA phosphoramidites DMT deprotection was performed with 3% (w/v) trichloroacetic acid in CH₂Cl₂ in three applications of 200 µL for 30 sec. The respective phosphoramidites were coupled three times with 100 µL of 0.1M solutions in acetonitrile (or acetonitrile/CH₂Cl₂ 1:1 for the LNA-^{Me}C building block) and 110 µL of a 0.1M solution of 5-(3,5-bis(trifluoromethylphenyl))-1H-tetrazole in acetonitrile as an activator and a coupling time of 180 sec. For thiooxidation a 0.1M solution of 3-amino-1,2,4-dithiazole-5-thione in acetonitrile/pyridine 1:1 was used (3x190 µL, 55 sec). Capping was performed using THF/lutidine/Ac₂O 8:1:1 (CapA, 75 µmol) and THF/N-methylimidazole 8:2 (CapB, 75 µmol) for 55 sec.

Synthesis cycles for the incorporation of 2',3'-dideoxy-3'-mercapto phosphoramidites included DMT deprotection using 3% (w/v) of trichloroacetic acid in CH₂Cl₂ in three applications of 200 µL for 30 sec. Phosphoramidite coupling was performed ten times with 40 µL of 0.1M solutions in acetonitrile and 44 µL of a 0.1M solution of 5-(3,5-bis(trifluoromethylphenyl))-1H-tetrazole in acetonitrile with a coupling time of 900 sec. Thiooxidation was performed immediately after coupling by applying three times 190 µL of a 0.1M solution of 3-amino-1,2,4-dithiazole-5-thione in acetonitrile/pyridine 1:1 for 55 sec. Capping was performed using THF/lutidine/Ac₂O 8:1:1 (CapA, 75 µmol) and THF/N-methylimidazole 8:2 (CapB, 75 µmol) for 55 sec.

Synthesis cycles for the incorporation of 2',5'-dideoxy-5'-mercapto phosphoramidites included coupling of the phosphoramidite building blocks using 100 µL of 0.1M solutions in acetonitrile and 110 µL of a 0.1M solution of 5-(3,5-bis(trifluoromethylphenyl))-1H-tetrazole in acetonitrile with a coupling time of 180 sec. Triple couplings were performed. Thiooxidation was performed using a 0.1M solution of 3-amino-1,2,4-dithiazole-5-thione in acetonitrile/pyridine 1:1 (3x190 µL, 55 sec). For capping, THF/lutidine/Ac₂O 8:1:1 (CapA, 75 µmol) and THF/N-methylimidazole 8:2 (CapB, 75 µmol) solutions were applied for 55 sec. DMT deprotection and liberation of the thiol was conducted with 3% (w/v) trichloroacetic acid in CH₂Cl₂ in 15 applications of 200 µL for 30 sec. DMT deprotection and liberation of the thiol was also advantageously conducted with 3-6 applications of 200 µL for 45 sec of 1-10% (v/v) trifluoroacetic acid or 5-10% (w/v) trichloroacetic acid, in the presence of 5-30% (v/v) triethylsilane in CH₂Cl₂ and/or 2-10% of *p*-methoxy thiophenol in CH₂Cl₂.

Removal of the nucleobase protecting groups and cleavage from the solid support was achieved under standard conditions using 32% aqueous ammonia at 55°C for a minimum of 8h. Crude DMT-on oligonucleotides were purified either using a solid phase extraction cartridge and repurification with ion exchange chromatography or by RP-HPLC purification using a C18 column followed by DMT removal with 80% aqueous acetic acid and ethanol precipitation.

According to the general procedures above the following molecules were prepared.

| Compound ID No. | Sequence | Calculated mass | Found mass |
|---|---|---|---|
| #1 | G^{m}C**at**tggtatT^{m}CA | 4357.6 | 4358.1 |
| #2 | G^{m}Ca**tt**ggtatT^{m}CA | 4357.6 | 4357.7 |
| #3 | G^{m}Cat**tg**gtatT^{m}CA | 4357.6 | 4357.4 |
| #4 | G^{m}Catt**gg**tatT^{m}CA | 4357.6 | 4357.6 |
| #5 | G^{m}Cattg**gt**atT^{m}CA | 4357.6 | 4358.1 |
| #6 | G^{m}Cattgg**ta**tT^{m}CA | 4357.6 | 4358.2 |
| #7 | G^{m}Cattggt**at**T^{m}CA | 4357.6 | 4358.0 |
| #8 | G^{m}Cattggta**tT**^{m}CA | 4357.6 | 4358.2 |

| | | | |
|---|---|---|---|
| Trithioate modifications between bold and underlined nucleotides A, G, ^{m}C, T represent LNA nucleotides a, g, c, t represent DNA nucleotides all other linkages were prepared as phosphorothioates | | | |

Compounds #1-8 are based on SEQ ID NO: 1. They differ in the position of the phosphorotrithioate internucleoside linkage of formula (I) as indicated in the above table.

### Example 2

### In vitro mRNA reduction and intracellular concentration (uptake)

Primary rat Hepatocytes were plated in 96-well plates and treated in Williams Medium E containing 10% FCS without antibiotics. Cells were treated with LNA solutions in the indicated concentrations in full cell culture medium. After an incubation time of 24 and 72 hrs, respectively, the cells were washed 3 times with PBS containing Ca²⁺ and Mg²⁺ and lysed with 165 uL PureLink Pro lysis buffer. Total RNA was isolated using the PureLink PRO 96 RNA Kit from Thermo Fisher according to the manufacturers instructions and RT-qPCR was performed using the LightCycler Multiplex RNA Virus Master (Roche) with Primer Probe Sets for RnApoB (Invitrogen). The obtained data was normalized to Ribogreen.

Intracellular concentrations of the LNA oligonucleotides were determined using an hybridization based ELISA assay. All data points were performed in duplicates and data is given as the average thereof.

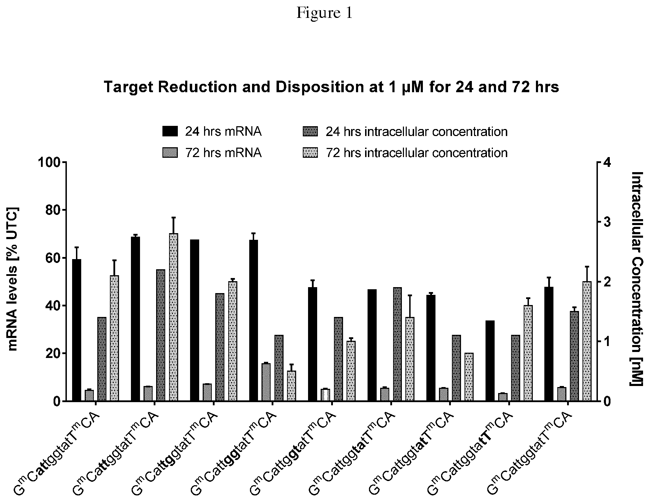

The results are given in Figure 1.

As can be seen from the data, the modification according to the invention is very well tolerated and the oligonucleotides show good potency as well as suitable intracellular concentrations.

### Example 3

### In vivo mRNA reduction in liver and kidney

C57BL/6JBomTac female mice at app 20 grams received a single dose at 1 mg/kg at a concentration of 0.1 mg/kg in a volume of 10 ml/kg day 0, the animals were terminated on day 7. Serum, liver-, kidney- and heart tissue was collected at termination.

mRNA analysis from tissue was performed using the Qantigene mRNA quantification kit ("bDNA-assay", Panomics/Affimetrix), following the manufacturers protocol. For tissue lysates, 50-80 mg of tissue was lysed by sonication in 1 ml lysis-buffer containing Proteinase K. Lysates were used directly for bDNA-assay without RNA extraction. Probesets for the target and GAPDH were obtained custom designed from Panomics. For analysis, luminescence units obtained for target genes were normalized to the housekeeper GAPDH.

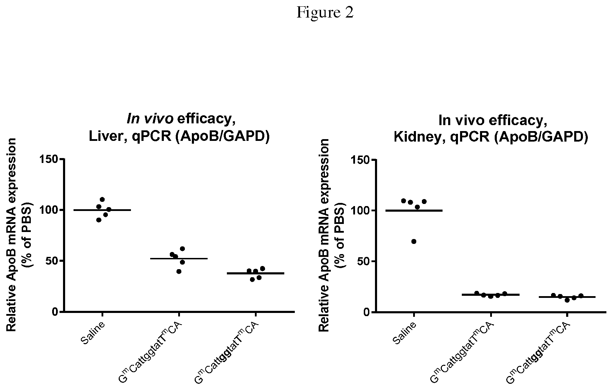

The results are given in Figure 2.

Figure 2 shows an improved target reduction in liver of the oligonucleotide bearing the modification according to the invention compared to an unmodified phosphorothioate control. These findings are even more surprising in light of the fact that this particular molecule demonstrated the lowest *in vitro* target reduction within the series.

### Example 4

### Serum cholesterol levels

C57BL/6JBomTac female mice at app 20 grams received a single dose at 1 mg/kg at a concentration of 0.1 mg/kg in a volume of 10 ml/kg day 0, the animals were terminated on day 7. Serum, liver-, kidney- and heart tissue was collected at termination.

Serum analysis for cholesterol was performed on the "Cobas INTEGRA 400 plus" clinical chemistry platform (Roche Diagnostics), using 10µl of serum.

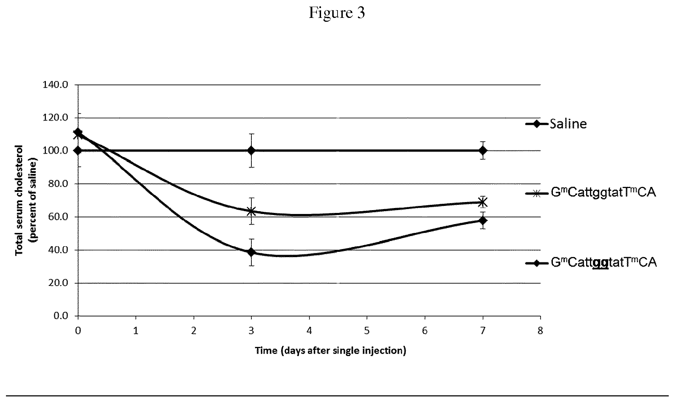

The results are given in Figure 3.

The data demonstrates that the observed higher target reduction of an oligonucleotide bearing the modification according to the invention (Figure 2, Example 3) results in an improved lowering of serum cholesterol levels, which is a functional readout of activity for this particular sequence. These findings are even more surprising in light of the fact that this particular molecule demonstrated the lowest *in vitro* target reduction within the series.

### Example 5

### In vivo tissue uptake in liver, kidney and heart

C57BL/6JBomTac female mice at app 20 grams received a single dose at 1 mg/kg at a concentration of 0.1 mg/kg in a volume of 10 ml/kg day 0, the animals were terminated on day 7. Serum, liver-, kidney- and heart tissue was collected at termination.

For oligonucleotide quantification, a fluorescently-labeled PNA probe is hybridized to the oligo of interest in the tissue lysate. The same lysates are used as for bDNA-assays, just with exactly weighted amounts of tissue. The heteroduplex is quantified using AEX-HPLC and fluorescent detection.

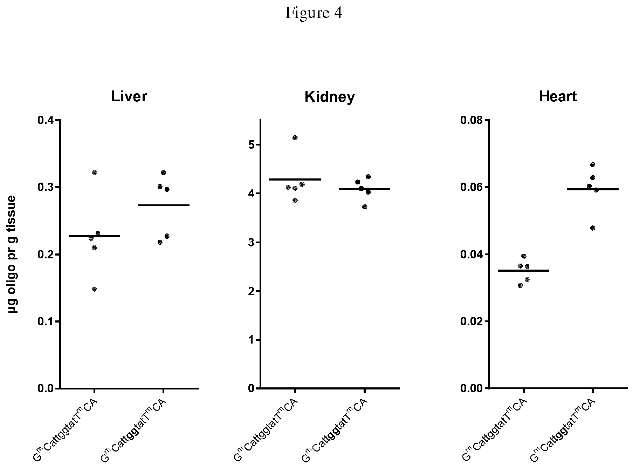

The results are given in Figure 4.

Figure 4 demonstrates demonstrates the beneficial effect of the modification according to the invention on tissue uptake. The data demonstrates not only a high tissue content in liver and kidney, but also the surprising potential of this modification to improve tissue distribution to the heart. These findings are even more surprising in light of the fact that this particular molecule demonstrated the lowest *in vitro* target reduction within the series.

## Claims

1. An oligonucleotide comprising at least one phosphorotrithioate internucleoside linkage of formula (I) 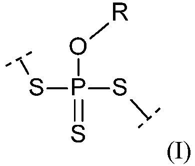 wherein one of the two bridging sulfur atoms is linked to the 3'carbon atom of a DNA nucleoside or a RNA nucleoside (A¹) and the other one is linked to the 5'carbon atom of another nucleoside (A²) and wherein R is hydrogen or a phosphate protecting group.

2. An oligonucleotide according to claim 1, wherein the nucleoside (A¹) is a DNA nucleoside.

3. An oligonucleotide according to claim 1 or 2, wherein the nucleoside (A²) is a DNA nucleoside, a RNA nucleoside or a sugar modified nucleoside.

4. An oligonucleotide according to any one of claims 1 to 3, wherein the nucleoside (A²) is a DNA nucleoside or a sugar modified nucleoside.

5. An oligonucleotide according to claim 3 or 4, wherein the sugar modified nucleoside is a 2' sugar modified nucleoside.

6. An oligonucleotide according to any one of claims 1 to 5, wherein the nucleoside (A²) is 2'-alkoxy-RNA, in particular 2'-methoxy-RNA, 2'-alkoxyalkoxy-RNA, in particular 2'-methoxyethoxy-RNA, 2'-amino-DNA, 2'-fluoro-RNA or 2'-fluoro-ANA.

7. An oligonucleotide according to any one of claims 1 to 5, wherein the nucleoside (A²) is a LNA nucleoside.

8. An oligonucleotide according to claim 7, wherein the LNA nucleoside is independently selected from beta-D-oxy LNA, 6'-methyl-beta-D-oxy LNA and ENA, in particular beta-D-oxy LNA.

9. An oligonucleotide according to claim 1, wherein at least one of the nucleosides (A¹) and (A²) is a 2'-alkoxyalkoxy-RNA.

10. An oligonucleotide according to claim 9, wherein the 2'-alkoxyalkoxy-RNA is 2'-methoxyethoxy-RNA.

11. An oligonucleotide according to any one of claims 1 to 4, wherein the nucleosides (A¹) and (A²) are both DNA nucleosides.

12. An oligonucleotide according to any one of claims 1 to 11, comprising further internucleoside linkages selected from phosphodiester internucleoside linkage, phosphorothioate internucleoside linkage and phosphorotrithioate internucleoside linkage of formula (I) as defined in claim 1.

13. An oligonucleotide according to any one of claims 1 to 12, comprising further internucleoside linkages selected from phosphorothioate internucleoside linkage and phosphorotrithioate internucleoside linkage of formula (I) as defined in claim 1.

14. An oligonucleotide according to any one of claims 1 to 13, comprising between 1 and 15, in particular between 1 and 5, more particularly 1, 2, 3, 4 or 5 phosphorotrithioate internucleoside linkages of formula (I) as defined in claim 1.

15. An oligonucleotide according to any one of claims 1 to 14, wherein the further internucleoside linkages are all phosphorothioate internucleoside linkages of formula - P(=S)(OR)O₂-, wherein R is as defined in claim 1.

16. An oligonucleotide according to any one of claims 1 to 15, comprising further nucleosides selected from DNA nucleoside, RNA nucleoside and sugar modified nucleosides.

17. An oligonucleotide according to any one of claims 1 to 16, wherein one or more nucleoside is a nucleobase modified nucleoside.

18. An oligonucleotide according to any one of claims 1 to 17, wherein the oligonucleotide is an antisense oligonucleotide, an siRNA, a microRNA mimic or a ribozyme.

19. An oligonucleotide according to any one of claims 1 to 18, wherein the oligonucleotide is an antisense gapmer oligonucleotide.

20. An oligonucleotide according to claim 19, wherein the phosphorotrithioate internucleoside linkage of formula (I) is in the gap region of the gapmer oligonucleotide.

21. An oligonucleotide according to claim 19 or 20, wherein the gapmer oligonucleotide is a LNA gapmer, a mixed wing gapmer or a 2'-substituted gapmer, in particular a 2'-O-methoxyethyl gapmer.

22. A gapmer oligonucleotide according to any one of claims 19 to 21, wherein the gapmer oligonucleotide comprises a contiguous nucleotide sequence of formula 5'-F-G-F'-3', wherein G is a region of 5 to 18 nucleosides which is capable of recruiting RnaseH, and said region G is flanked 5' and 3' by flanking regions F and F' respectively, wherein regions F and F' independently comprise or consist of 1 to 7 2'-sugar modified nucleotides, wherein the nucleoside of region F which is adjacent to region G is a 2'-sugar modified nucleoside and wherein the nucleoside of region F' which is adjacent to region G is a 2'-sugar modified nucleoside.

23. A gapmer oligonucleotide according to claim 22, wherein said at least one phosphorotrithioate internucleoside linkage of formula (I) as defined in claim 1 is positioned between adjacent nucleosides in region G or between region G and region F'.

24. An oligonucleotide according to any one of claims 1 to 23, wherein the oligonucleotide is an antisense oligonucleotide mixmer or totalmer, in particular a splice-switching oligonucleotide or a microRNA inhibitor oligonucleotide.

25. A pharmaceutically acceptable salt of an oligonucleotide according to any one of claims 1 to 24, in particular a sodium or a potassium salt.

26. A conjugate comprising an oligonucleotide or a pharmaceutically acceptable salt according to any one of claims 1 to 25 and at least one conjugate moiety covalently attached to said oligonucleotide or said pharmaceutically acceptable salt, optionally *via* a linker moiety.

27. A pharmaceutical composition comprising an oligonucleotide, a pharmaceutically acceptable salt or a conjugate according to any one of claims 1 to 26 and a therapeutically inert carrier.

28. An oligonucleotide, pharmaceutically acceptable salt or conjugate according to any one of claims 1 to 26 for use as therapeutically active substance.

29. An oligonucleotide, pharmaceutically acceptable salt or conjugate according to any one of claims 1 to 26 for use in the treatment or prophylaxis of a heart or blood disease.

30. A process for the manufacture of an oligonucleotide according to any one of claims 1 to 24 comprising the following steps:
(a) Coupling a 3'S-modified nucleoside phosphoramidite to the terminal 5' sulfur atom of a 5'S- modified nucleoside or oligonucleotide to produce a dithiophosphite triester intermediate;
(b) Thiooxidizing the dithiophosphite triester intermediate obtained in step a); and
(c) Optionally further elongating the oligonucleotide.

## Patentansprüche

1. Oligonukleotid, umfassend mindestens eine Phosphortrithioat-InternukleosidVerknüpfung der Formel (I) 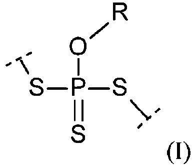 wobei eines der zwei Brückenschwefelatome mit dem 3'-Kohlenstoffatom eines DNA-Nukleosids oder eines RNA-Nukleosids (A¹) verknüpft ist und das andere mit dem 5'-Kohlenstoffatom eines anderen Nukleosids (A²) verknüpft ist und wobei R Wasserstoff oder eine Phosphatschutzgruppe ist.

2. Oligonukleotid nach Anspruch 1, wobei das Nukleosid (A¹) ein DNA-Nukleosid ist.

3. Oligonukleotid nach Anspruch 1 oder 2, wobei das Nukleosid (A²) ein DNA-Nukleosid, ein RNA-Nukleosid oder ein zuckermodifiziertes Nukleosid ist.

4. Oligonukleotid nach einem der Ansprüche 1 bis 3, wobei das Nukleosid (A²) ein DNA-Nukleosid oder ein zuckermodifiziertes Nukleosid ist.

5. Oligonukleotid nach Anspruch 3 oder 4, wobei das zuckermodifizierte Nukleosid ein 2'-zuckermodifiziertes Nukleosid ist.

6. Oligonukleotid nach einem der Ansprüche 1 bis 5, wobei das Nukleosid (A²) 2'-Alkoxy-RNA, insbesondere 2'-Methoxy-RNA, 2'-Alkoxyalkoxy-RNA, insbesondere 2'-Methoxyethoxy-RNA, 2'-Amino-DNA, 2'-Fluor-RNA oder 2'-Fluor-ANA ist.

7. Oligonukleotid nach einem der Ansprüche 1 bis 5, wobei das Nukleosid (A²) ein LNA-Nukleosid ist.

8. Oligonukleotid nach Anspruch 7, wobei das LNA-Nukleosid unabhängig ausgewählt ist aus beta-D-Oxy-LNA, 6'-Methyl-beta-D-oxy-LNA und ENA, insbesondere beta-D-Oxy-LNA.

9. Oligonukleotid nach Anspruch 1, wobei mindestens eines der Nukleoside (A¹) und (A²) eine 2'-Alkoxyalkoxy-RNA ist.

10. Oligonukleotid nach Anspruch 9, wobei die 2'-Alkoxyalkoxy-RNA 2'-Methoxyethoxy-RNA ist.

11. Oligonukleotid nach einem der Ansprüche 1 bis 4, wobei die Nukleoside (A¹) und (A²) beide DNA-Nukleoside sind.

12. Oligonukleotid nach einem der Ansprüche 1 bis 11, umfassend weitere Internukleosidverknüpfungen, ausgewählt aus Phosphodiester-Internukleosidverknüpfung, Phosphorthioat-Internukleosidverknüpfung und Phosphortrithioat-Internukleosidverknüpfung der Formel (I), wie in Anspruch 1 definiert.

13. Oligonukleotid nach einem der Ansprüche 1 bis 12, umfassend weitere Internukleosidverknüpfungen, ausgewählt aus Phosphorthioat-Internukleosidverknüpfung und Phosphortrithioat-Internukleosidverknüpfung der Formel (I), wie in Anspruch 1 definiert.

14. Oligonukleotid nach einem der Ansprüche 1 bis 13, umfassend zwischen 1 und 15, insbesondere zwischen 1 und 5, speziell 1, 2, 3, 4 oder 5 Phosphortrithioat-Internukleosidverknüpfungen der Formel (I), wie in Anspruch 1 definiert.

15. Oligonukleotid nach einem der Ansprüche 1 bis 14, wobei die weiteren Internukleosidverknüpfungen alle Phosphorthioat-Internukleosidverknüpfungen der Formel -P(=S)(OR)O₂- sind, wobei R wie in Anspruch 1 definiert ist.

16. Oligonukleotid nach einem der Ansprüche 1 bis 15, umfassend weitere Nukleoside, ausgewählt aus DNA-Nukleosid, RNA-Nukleosid und zuckermodifizierten Nukleosiden.

17. Oligonukleotid nach einem der Ansprüche 1 bis 16, wobei ein oder mehrere Nukleoside ein nukleobasenmodifiziertes Nukleosid sind.

18. Oligonukleotid nach einem der Ansprüche 1 bis 17, wobei das Oligonukleotid ein Antisense-Oligonukleotid, eine siRNA, ein microRNA-Imitator oder ein Ribozym ist.

19. Oligonukleotid nach einem der Ansprüche 1 bis 18, wobei das Oligonukleotid ein Antisense-Gapmer-Oligonukleotid ist.

20. Oligonukleotid nach Anspruch 19, wobei die Phosphortrithioat-Internukleosidverknüpfung der Formel (I) in der Gap-Region des Gapmer-Oligonukleotids liegt.

21. Oligonukleotid nach Anspruch 19 oder 20, wobei das Gapmer-Oligonukleotid ein LNA-Gapmer, ein Mixed-Wing-Gapmer oder ein 2'-substituiertes Gapmer, insbesondere ein 2'-O-Methoxyethyl-Gapmer, ist.

22. Gapmer-Oligonukleotid nach einem der Ansprüche 19 bis 21, wobei das Gapmer-Oligonukleotid eine zusammenhängende Nukleotidsequenz der Formel 5'-F-G-F'-3' umfasst, wobei G eine Region von 5 bis 18 Nukleosiden ist, die in der Lage ist, RnaseH zu rekrutieren, und die Region G 5' und 3' durch flankierende Regionen F bzw. F' flankiert ist, wobei die Regionen F und F' unabhängig voneinander 1 bis 7 2'-zuckermodifizierte Nukleotide umfassen oder daraus bestehen, wobei das Nukleosid der Region F, das an die Region G angrenzt, ein 2'-zuckermodifiziertes Nukleosid ist und wobei das Nukleosid der Region F', das an die Region G angrenzt, ein 2'-zuckermodifiziertes Nukleosid ist.

23. Gapmer-Oligonukleotid nach Anspruch 22, wobei die mindestens eine Phosphortrithioat-Internukleosidverknüpfung der Formel (I), wie in Anspruch 1 definiert, zwischen benachbarten Nukleosiden in der Region G oder zwischen der Region G und der Region F' positioniert ist.

24. Oligonukleotid nach einem der Ansprüche 1 bis 23, wobei das Oligonukleotid ein Antisense-Oligonukleotid-Mixmer oder -Totalmer, insbesondere ein Spleiß-Switching-Oligonukleotid oder ein microRNA-Inhibitor-Oligonukleotid, ist.

25. Pharmazeutisch verträgliches Salz eines Oligonukleotids nach einem der Ansprüche 1 bis 24, insbesondere ein Natrium- oder ein Kaliumsalz.

26. Konjugat, umfassend ein Oligonukleotid oder ein pharmazeutisch verträgliches Salz nach einem der Ansprüche 1 bis 25 und mindestens eine Konjugateinheit, die kovalent an das Oligonukleotid oder das pharmazeutisch verträgliche Salz, gegebenenfalls über eine Linkereinheit, gebunden ist.

27. Pharmazeutische Zusammensetzung, umfassend ein Oligonukleotid, ein pharmazeutisch verträgliches Salz oder ein Konjugat nach einem der Ansprüche 1 bis 26 und einen therapeutisch inerten Träger.

28. Oligonukleotid, pharmazeutisch verträgliches Salz oder Konjugat nach einem der Ansprüche 1 bis 26 zur Verwendung als therapeutisch aktive Substanz.

29. Oligonukleotid, pharmazeutisch verträgliches Salz oder Konjugat nach einem der Ansprüche 1 bis 26 zur Verwendung bei der Behandlung oder Prophylaxe einer Herz- oder Bluterkrankung.

30. Verfahren zur Herstellung eines Oligonukleotids nach einem der Ansprüche 1 bis 24, umfassend die folgenden Schritte:
(a) Kuppeln eines 3'S-modifizierten Nukleosidphosphoramidits an das terminale 5'-Schwefelatom eines 5'S-modifizierten Nukleosids oder Oligonukleotids, um ein Dithiophosphittriesterzwischenprodukt herzustellen;
(b) Thiooxidieren des in Schritt a) erhaltenen Dithiophosphittriesterzwischenprodukts; und
(c) gegebenenfalls weiteres Verlängern des Oligonukleotids.

## Revendications

1. Oligonucléotide comprenant au moins une liaison internucléosidique phosphorotrithioate de formule (I) 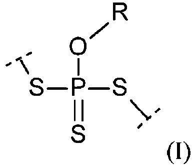 dans lequel l'un des deux atomes de soufre pontant est lié à l'atome de carbone en 3' d'un nucléoside d'ADN ou d'un nucléoside d'ARN (A¹) et l'autre est lié à l'atome de carbone en 5' d'un autre nucléoside (A²) et dans lequel R représente un hydrogène ou un groupe protecteur de phosphate.

2. Oligonucléotide selon la revendication 1, dans lequel le nucléoside (A¹) est un nucléoside d'ADN.

3. Oligonucléotide selon la revendication 1 ou 2, dans lequel le nucléoside (A²) est un nucléoside d'ADN, un nucléoside d'ARN ou un nucléoside à sucre modifié.

4. Oligonucléotide selon l'une quelconque des revendications 1 à 3, dans lequel le nucléoside (A²) est un nucléoside d'ADN ou un nucléoside à sucre modifié.

5. Oligonucléotide selon la revendication 3 ou 4, dans lequel le nucléoside à sucre modifié est un nucléoside à sucre modifié en 2'.

6. Oligonucléotide selon l'une quelconque des revendications 1 à 5, dans lequel le nucléoside (A²) est un 2'-alcoxy-ARN, en particulier un 2'-méthoxy-ARN, un 2'-alcoxyalcoxy-ARN, en particulier un 2'-méthoxyéthoxy-ARN, un 2'-amino-ADN, un 2'-fluoro-ARN ou un 2'-fluoro-ANA.

7. Oligonucléotide selon l'une quelconque des revendications 1 à 5, dans lequel le nucléoside (A²) est un nucléoside de LNA.

8. Oligonucléotide selon la revendication 7, dans lequel le nucléoside de LNA est indépendamment choisi parmi un bêta-D-oxy LNA, un 6'-méthyl-bêta-D-oxy LNA et un ENA, en particulier un bêta-D-oxy LNA.

9. Oligonucléotide selon la revendication 1, dans lequel au moins l'un des nucléosides (A¹) et (A²) est un 2'-alcoxyalcoxy-ARN.

10. Oligonucléotide selon la revendication 9, dans lequel le 2'-alcoxyalcoxy-ARN est un 2'-méthoxyéthoxy-ARN.

11. Oligonucléotide selon l'une quelconque des revendications 1 à 4, dans lequel les nucléosides (A¹) et (A²) sont tous les deux des nucléosides d'ADN.

12. Oligonucléotide selon l'une quelconque des revendications 1 à 11, comprenant d'autres liaisons internucléosidiques choisies parmi une liaison internucléosidique phosphodiester, une liaison internucléosidique phosphorothioate et une liaison internucléosidique phosphorotrithioate de formule (I) telle que définie dans la revendication 1.

13. Oligonucléotide selon l'une quelconque des revendications 1 à 12, comprenant d'autres liaisons internucléosidiques choisies parmi une liaison internucléosidique phosphorothioate et une liaison internucléosidique phosphorotrithioate de formule (I) telle que définie dans la revendication 1.

14. Oligonucléotide selon l'une quelconque des revendications 1 à 13, comprenant entre 1 et 15, en particulier entre 1 et 5, plus particulièrement 1, 2, 3, 4 ou 5 liaisons internucléosidiques phosphorotrithioate de formule (I) telle que définie dans la revendication 1.

15. Oligonucléotide selon l'une quelconque des revendications 1 à 14, dans lequel les autres liaisons internucléosidiques sont toutes des liaisons internucléosidiques phosphorothioate de formule -P(=S)(OR)O₂-, dans lequel R est tel que défini dans la revendication 1.

16. Oligonucléotide selon l'une quelconque des revendications 1 à 15, comprenant d'autres nucléosides choisis parmi un nucléoside d'ADN, un nucléoside d'ARN et des nucléosides à sucre modifié.

17. Oligonucléotide selon l'une quelconque des revendications 1 à 16, dans lequel un ou plusieurs nucléosides sont un nucléoside à nucléobase modifiée.

18. Oligonucléotide selon l'une quelconque des revendications 1 à 17, dans lequel l'oligonucléotide est un oligonucléotide antisens, un pARNi, un mimétique de microARN ou un ribozyme.

19. Oligonucléotide selon l'une quelconque des revendications 1 à 18, dans lequel l'oligonucléotide est un oligonucléotide gapmère antisens.

20. Oligonucléotide selon la revendication 19, dans lequel la liaison internucléosidique phosphorotrithioate de formule (I) se trouve dans la région d'espace de l'oligonucléotide gapmère.

21. Oligonucléotide selon la revendication 19 ou 20, dans lequel l'oligonucléotide gapmère est un gapmère de LNA, un gapmère à ailes mixtes ou un gapmère substitué en 2', en particulier un gapmère 2'-O-méthoxyéthylique.

22. Oligonucléotide gapmère selon l'une quelconque des revendications 19 à 21, dans lequel l'oligonucléotide gapmère comprend une séquence nucléotidique contiguë de formule 5'-F-G-F'-3', dans lequel G représente une région de 5 à 18 nucléosides qui est capable de recruter la RNase H, et ladite région G est encadrée en 5' et 3' respectivement par les régions d'encadrement F et F', dans lequel les régions F et F' comprennent ou sont constituées indépendamment de 1 à 7 nucléotides à sucre modifié en 2', dans lequel le nucléoside de région F qui est adjacent à la région G est un nucléoside à sucre modifié en 2' et dans lequel le nucléoside de la région F' qui est adjacent à la région G est un nucléoside à sucre modifié en 2'.

23. Oligonucléotide gapmère selon la revendication 22, dans lequel ladite au moins une liaison internucléosidique phosphorotrithioate de formule (I) telle que définie dans la revendication 1 est positionnée entre des nucléosides adjacents dans la région G ou entre la région G et la région F'.

24. Oligonucléotide selon l'une quelconque des revendications 1 à 23, dans lequel l'oligonucléotide est un mixmère ou un totalmère d'oligonucléotide antisens, en particulier un oligonucléotide de commutation d'épissage ou un oligonucléotide inhibiteur de microARN.

25. Sel pharmaceutiquement acceptable d'un oligonucléotide selon l'une quelconque des revendications 1 à 24, en particulier un sel de sodium ou de potassium.

26. Conjugué comprenant un oligonucléotide ou un sel pharmaceutiquement acceptable selon l'une quelconque des revendications 1 à 25 et au moins une fraction conjuguée liée de manière covalente audit oligonucléotide ou audit sel pharmaceutiquement acceptable, éventuellement par l'intermédiaire d'une fraction de liaison.

27. Composition pharmaceutique comprenant un oligonucléotide, un sel pharmaceutiquement acceptable ou un conjugué selon l'une quelconque des revendications 1 à 26 et un véhicule thérapeutiquement inerte.

28. Oligonucléotide, sel pharmaceutiquement acceptable ou conjugué selon l'une quelconque des revendications 1 à 26 pour une utilisation comme substance thérapeutiquement active.

29. Oligonucléotide, sel pharmaceutiquement acceptable ou conjugué selon l'une quelconque des revendications 1 à 26 pour une utilisation dans le traitement ou la prophylaxie d'une maladie du cœur ou du sang.

30. Procédé de fabrication d'un oligonucléotide selon l'une quelconque des revendications 1 à 24 comprenant les étapes suivantes :
(a) couplage d'un nucléoside phosphoramidite modifié par un S en 3' à l'atome de soufre terminal en 5' d'un nucléoside ou d'un oligonucléotide modifié par un S en 5' pour produire un intermédiaire dithiophosphite triester ;
(b) thiooxydation de l'intermédiaire dithiophosphite triester obtenu à l'étape a) ; et
(c) éventuellement allongement supplémentaire de l'oligonucléotide.
